# EUROPEAN PATENT SPECIFICATION

(11) **EP 4 208 234 B1**
(45) Date of publication and mention of the grant of the patent: **08.04.2026**
(21) Application number: 21773301.3
(22) Date of filing: 31.08.2021
(51) Int. Cl.: A61M 15/08, A61M 11/06, A61M 15/00

(54) **DRUG DELIVERY DEVICES WITH MULTIPLE DRUG VIALS**
ARZNEIMITTELABGABEVORRICHTUNGEN MIT MEHREREN ARZNEIMITTELAMPULLEN
DISPOSITIFS D'ADMINISTRATION DE MÉDICAMENT AVEC MULTIPLES FLACONS DE MÉDICAMENT

(30) Priority: 01.09.2020 US 202063073281 P
(43) Date of publication of application: 12.07.2023
(60) Divisional application: 25212695.8
(73) Proprietor: Janssen Pharmaceutica NV, 2340 Beerse (BE)
(72) Inventor: SHEPHERD, David, Edinburgh EH12 7HW (GB); MARTIN, Scott, Edinburgh EH6 6LA (GB); CANNAMELA, Michael, New York 10001 (US); CASSEBEE, Jimmy Vinh Hoang, Milpitas, California 95035 (US); YAN, Hong, Milpitas, California 95035 (US); PÉREZ, Dolores, Easton, Pennsylvania 18045 (US); POPLI, Shagun, Milpitas, California 95035 (US); HUBERT, Emma Louise, Milpitas, California 95035 (US); VESOLE, Steven M., Milpitas, California 95035 (US)
(74) Representative: Carpmaels & Ransford LLP
(86) International application number: PCT/EP2021/074055
(87) International publication number: WO 2022/049087

(56) References cited:
- WO-A2-02/13886
- WO-A2-2005/102058
- US-A- 5 944 222
- US-A1- 2010 258 118

## Description

### FIELD

The present disclosure relates generally to drug delivery devices with multiple drug vials and drug products utilizing the same.

### BACKGROUND

There are many different ways in which a drug can be administered to a user. Depending on the drug, intranasal drug delivery can be one of the most effective ways to achieve desired clinical benefits in a timely manner and in a manner that is convenient and comfortable for a patient.

Intranasal drug administration is a non-invasive route for drug delivery. Since the nasal mucosa offers numerous benefits as a target tissue for drug delivery, a wide variety of drugs may be administered by intranasal systemic action. Moreover, intranasal drug delivery can avoid the risks and discomfort associated with other routes of drug delivery, such as intravenous drug delivery, and can allow for easy self-administration.

Generally, to maximize the efficacy of the drug through intranasal administration, the majority volume of the aerosolized dose of the drug needs to reach the correct region of the nasal cavity. As such, additional measures may need to be taken for effective intranasal drug delivery. For example, the user may need to have a clear nostril, tilt their head back at approximately 45°, close the opposite nostril, and then sniff gently while the dose of drug is administered. In order to coordinate these measures, and given that nasal administration is intimate, self-administration by the user may be desired. Further, due to the nasal cycle (alternating physiological partial congestion of the nasal turbinate to facilitate nasal function) or pathological congestion, one nostril is likely to provide a more effective drug delivery route than the other nostril at any given time. As such, it is desired that an equal dose of the drug be delivered to each nostril of the user to inhibit under-dosing of the drug.

Dual-dose intranasal drug delivery devices are available that are designed for self-administration of two distinct aerosolized sprays, one for each nostril, that together constitute one dose of drug. These devices require a series of operational steps that the user needs to properly carry out to effect optimal drug delivery through self-administration. After the drug is delivered into each nostril, the device may be disposed of as a used device having no drug remaining in the device or having a residual amount of the drug remaining in the device that cannot be further delivered from the device. Disposing the device after use may have adverse environmental impacts by generating waste.

Some nasal drug delivery devices are designed for one-time use to deliver one dose of drug, such as for prescription drugs where the amount of drug delivered to the patient is important and is typically prescribed at a certain amount per dose. Such one-time use devices may therefore have exacerbated adverse environmental impacts since they are only used for one drug spray before disposal. Additionally, one-time use nasal drug delivery devices prevent patients from becoming familiar and comfortable with using the same device for multiple drug deliveries. Even if each sequentially used nasal drug delivery device is the same as one another, at least some patients may not recognize each device's similarity and/or may feel reluctance and/or nervousness at using a device they have never used before. WO2005102058A2 describes a multiple unit dose drug delivery system. WO0213886A2 describes a programmable multi-dose intranasal drug delivery device. US2010258118A1 describes inhaler devices and bespoke pharmaceutical compositions.

Accordingly, there remains a need for improved nasal drug delivery devices.

### SUMMARY

In general, drug delivery devices with multiple drug vials, drug products utilizing the same, and methods of using drug delivery devices with multiple drug vials are provided. The invention is defined by the appended claims.

In one aspect, a drug delivery device is provided that in one embodiment includes a tip configured to be positioned in a nose of a patient. The tip has an opening therein. The drug delivery device also includes a cartridge configured to seat a plurality of vials that each contain a drug therein. A first one of the vials is aligned with the tip. The drug delivery device also includes a plunger configured to actuated to cause drug in a first one of the vials to be delivered through the opening and to cause the cartridge to rotate relative to the tip and to the plunger such that the first one of the vials is misaligned from the tip and a second one of the vials is aligned with the tip.

The drug delivery device can vary in any number of ways. For example, the plurality of vials can include a total of six vials. For another example, the plurality of vials can include only the first and second vials. For yet another example, the actuation of the plunger can include moving the plunger proximally relative to the tip, and the drug delivery device can also include a spring configured to compress during the proximal movement of the tip and to automatically expand to cause the plunger to move distally relative to the tip.

For still another example, the drug delivery device can include a body that includes a protrusion, and the cartridge can have a groove formed therein in which the protrusion is configured to slide to cause the rotation of the cartridge about a longitudinal axis of the cartridge. The actuation of the plunger can include moving the plunger longitudinally relative to the tip and the body. A longitudinal axis of the tip can be offset from the longitudinal axis of the cartridge. The body can include a window therein, and the cartridge can include a plurality of indicators thereon that are configured to be visible sequentially through the window. The rotation of the cartridge can cause a one of the indictors that is visible through the window to change to another one of the indicators.

For another example, with the second one of the vials aligned with the tip, the plunger can be configured to be actuated again to cause drug in the second one of the vials to be delivered through the opening and to cause the cartridge to rotate relative to the tip and to the plunger. The rotation of the cartridge can cause the second one of the vials to become misaligned from the tip and a third one of the vials to be aligned with the tip, or the plunger can be configured to not be actuated again after the drug in the second one of the vials is delivered through the opening.

For yet another example, the drug delivery device can include the plurality of vials that each contain the drug therein. For still another example, the drug in each of the vials is one of ketamine, esketamine, naloxone, and sumatriptan.

For another example, a drug delivery method can include actuating the plunger of the drug delivery device. The drug in each of the vials is one of ketamine, esketamine, naloxone, and sumatriptan.

**In** another aspect, a drug product is provided that in one embodiment includes a drug product disposed in a drug delivery device. The drug delivery device includes a tip configured to be positioned in a nose of a patient. The tip has an opening therein. The drug delivery device also includes a cartridge configured to seat a plurality of vials that each contain the drug product therein. A first one of the vials is aligned with the tip. The drug delivery device also includes a plunger configured to actuated to cause drug product in a first one of the vials to be delivered through the opening and to cause the cartridge to rotate relative to the tip and to the plunger such that the first one of the vials is misaligned from the tip a second one of the vials is aligned with the tip. The drug product is one of ketamine, esketamine, naloxone, and sumatriptan. The drug delivery device can have any number of variations.

**In** another embodiment, a drug delivery device is provided that in one embodiment includes a tip configured to be positioned in a nose of a patient. The tip has an opening therein. The drug delivery device also includes a drug holder configured to hold a single vial that contains a drug therein, a plunger configured to be actuated to cause a partial amount of the drug in the vial to be delivered through the opening, and a ratchet mechanism configured to cause the drug holder to move from a first position relative to the tip before the actuation of the plunger to a second position relative to the tip after the partial amount of the drug has been delivered through the opening.

The drug delivery device can vary in any number of ways. For example, the actuation of the plunger can be configured to move the plunger proximally relative to the tip, and the second position can be proximal to the first position. For another example, the plunger can be configured to be actuated only one more time to cause substantially all of a remainder of the drug in the vial to be delivered through the opening, and the ratchet mechanism can be configured to cause the drug holder to move from the second position relative to the tip before the one more actuation of the plunger to a third position relative to the tip after the substantially all of the remainder of the drug has been delivered through the opening. For yet another example, the plunger can be configured to be actuated a plurality of additional times to each cause a partial amount of the drug in the vial to be delivered through the opening, and the ratchet mechanism can be configured to cause the drug holder to move to a different position relative to the tip after each additional actuation.

For another example, the ratchet mechanism can include a plurality of teeth formed on an outer surface of the drug holder. Each of the teeth can be at a different axial position along a longitudinal length of the drug holder. The ratchet mechanism can also include a surface of the plunger. The drug delivery device can also include a collar, the actuation of the plunger can be configured to cause the collar to rotate relative to the drug holder, and the collar can include a plurality of indicators thereon configured to indicate visually a number of remaining times that the plunger can be actuated to deliver the drug through the opening.

For still another example, the ratchet mechanism can include a tab extending radially outward from the drug holder. The drug delivery device can also include a collar, and the ratchet mechanism can also include a plurality of ratchet steps formed in the collar. The actuation of the plunger can be configured to cause the collar to rotate relative to the drug holder. The rotation of the collar can cause the tab to move from one of the ratchet steps to another one of the ratchet steps. The collar can include a plurality of indicators thereon configured to indicate visually a number of remaining times that the plunger can be actuated to deliver the drug through the opening.

For yet another example, the drug delivery device can include the vial. For another example, the drug can be one of ketamine, esketamine, naloxone, and sumatriptan.

For still another example, a drug delivery method can include actuating the plunger of the drug delivery device. The drug can be one of ketamine, esketamine, naloxone, and sumatriptan.

**In** another aspect, a drug product is provided that in one embodiment includes a drug product disposed in a drug delivery device. The drug delivery device includes a tip configured to be positioned in a nose of a patient. The tip has an opening therein. The drug delivery device also includes a drug holder configured to hold a single vial that contains the drug product therein, a plunger configured to actuated to cause a partial amount of the drug product in the vial to be delivered through the opening, and a ratchet mechanism configured to cause the drug holder to move from a first position relative to the tip before the actuation of the plunger to a second position relative to the tip after the partial amount of the drug product has been delivered through the opening. The drug product is one of ketamine, esketamine, naloxone, and sumatriptan. The drug delivery device can have any number of variations.

### BRIEF DESCRIPTION OF DRAWINGS

The present invention is described by way of reference to the accompanying figures which are as follows:
FIG. 1 is a block diagram of one embodiment of a drug delivery device;
FIG. 2 is a side cross-sectional view of one embodiment of a vial;
FIG. 3 is a perspective view of one embodiment of the drug delivery device of FIG. 1;
FIG. 4 is a cross-sectional view of the drug delivery device of FIG. 3;
FIG. 5 is an exploded view of the drug delivery device of FIG. 3;
FIG. 6 is another cross-sectional view of the drug delivery device of FIG. 3;
FIG. 7 is a yet another cross-sectional view of the drug delivery device of FIG. 3 with the drug delivery device in an actuated configuration;
FIG. 8 is a partially transparent view of the drug delivery device of FIG. 3 with the drug delivery device in an initial configuration;
FIG. 9 is a perspective cross-sectional view of another embodiment of the drug delivery device of FIG. 1;
FIG. 10 is a cross-sectional view of the drug delivery device of FIG. 9 after the drug delivery device has delivered one drug spray;
FIG. 11 is a cross-sectional view of the drug delivery device of FIG. 9 after the drug delivery device has delivered six drug sprays;
FIG. 12 is a partially transparent view of the drug delivery device of FIG. 9 after the drug delivery device has delivered two drug sprays;
FIG. 13 is an exploded view of yet another embodiment of the drug delivery device of FIG. 1;
FIG. 14 is a side cross-sectional view of the drug delivery device of FIG. 13;
FIG. 15 is a partially transparent view of the drug delivery device of FIG. 13 with the drug delivery device in an initial configuration;
FIG. 16 is a perspective view of a portion of the drug delivery device of FIG. 15 after the drug delivery device has delivered one drug spray;
FIG. 17 is a cross-sectional view of the drug delivery device of FIG. 15 after the drug delivery device has delivered six drug sprays;
FIG. 18 is a perspective view of another embodiment of the drug delivery device of FIG. 1;
FIG. 19 is a side cross-sectional view of the drug delivery device of FIG. 18;
FIG. 20 is a side partial view of another embodiment of the drug delivery device of FIG. 1;
FIG. 21 is a perspective view of a split plunger and a portion of a body of another embodiment of the drug delivery device of FIG. 1;
FIG. 22 is a side partial view of yet another embodiment of the drug delivery device of FIG. 1; and
FIG. 23 is a side partial view of still another embodiment of the drug delivery device of FIG. 1.

### DETAILED DESCRIPTION

Certain exemplary embodiments will now be described to provide an overall understanding of the principles of the structure, function, manufacture, and use of the devices, systems, and methods disclosed herein. One or more examples of these embodiments are illustrated in the accompanying drawings. A person skilled in the art will understand that the devices, systems, and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments. Such modifications and variations are intended to be included within the scope of the present invention.

Further, in the present disclosure, like-named components of the embodiments generally have similar features, and thus within a particular embodiment each feature of each like-named component is not necessarily fully elaborated upon. Additionally, to the extent that linear or circular dimensions are used in the description of the disclosed systems, devices, and methods, such dimensions are not intended to limit the types of shapes that can be used in conjunction with such systems, devices, and methods. A person skilled in the art will recognize that an equivalent to such linear and circular dimensions can easily be determined for any geometric shape. A person skilled in the art will appreciate that a dimension may not be a precise value but nevertheless be considered to be at about that value due to any number of factors such as manufacturing tolerances and sensitivity of measurement equipment. Sizes and shapes of the systems and devices, and the components thereof, can depend at least on the size and shape of components with which the systems and devices will be used.

Various exemplary drug delivery devices with multiple drug vials, drug products utilizing the same, and methods of using drug delivery devices with multiple drug vials are provided. **In** general, a nasal drug delivery device is configured to dispense a drug therefrom into a nose and includes two or more vials each containing the drug therein. The drug delivery device is configured to deliver the drug from each of the vials for a total of two or more sprays into the nose. The same drug delivery device can therefore deliver multiple drug sprays to a user, which may help improve user familiarity and comfort with the drug delivery device and/or may reduce waste compared to one-time use drug delivery devices since the drug delivery device is reusable for multiple drug sprays.

The multiple vials can be pre-loaded into the drug delivery device, such as by a manufacturer and/or a health care professional, before drug is delivered to a user from any of the vials, which may ease use of the drug delivery device since the user need not learn how to load vials into the drug delivery device, may help ensure that the vials are loaded properly into the drug delivery device by a trained, experienced party and that drug is consequently properly delivered to the user from the drug delivery device, and/or may help prevent unauthorized access to the drug in any of the vials as compared to drugs in vials that are not pre-loaded into a drug delivery device. Preventing unauthorized access to a drug may be particularly important for esketamine, ketamine, and other controlled substances.

Some drug doses may require only one drug spray. For such drug doses, the drug delivery device allows for the same drug delivery device to be used multiple times to deliver multiple doses, which may help improve user familiarity and comfort with the drug delivery device and/or may generate less waste than nasal drug delivery devices that are disposed of after delivering one drug spray therefrom. Some drug doses may require two drug sprays, e.g., one spray into each nostril. For such drug doses, the drug delivery device being configured to deliver more than two drug sprays allows for the same drug delivery device to be used multiple times to deliver multiple doses, which may help improve user familiarity and comfort with the drug delivery device and/or may generate less waste than nasal drug delivery devices that are disposed of after delivering one drug spray therefrom. Some drug doses may require two drug sprays, e.g., one spray into each nostril. Some drug doses may require six drug sprays, e.g., three sprays into each nostril. For such drug doses, the drug delivery device being configured to deliver at least six sprays allows the same drug delivery device to be used to deliver the complete drug dose, unlike traditional nasal drug delivery devices that can only deliver one or two drug sprays therefrom. The drug delivery device may thus help improve user familiarity and comfort with the drug delivery device and/or may generate less waste than nasal drug delivery devices that are disposed of after delivering one or two drug sprays therefrom as part of a user's six-spray drug dose.

Some vials contain only enough drug therein for one drug spray, e.g., to ease manufacturing, to limit access to the drug, etc. The drug delivery device can include two or more vials each containing only enough drug therein for one drug spray, thereby allowing the drug delivery device to be used for multiple drug sprays. Some vials contain only enough drug therein for two drug sprays, e.g., to ease manufacturing, to limit access to the drug, etc. The drug delivery device can include more than two vials each containing only enough drug therein for two drug sprays, thereby allowing the drug delivery device to be used for at least four drug sprays.

In some embodiments, instead of including multiple vials that collectively contain enough drug therein for more than two drug sprays, the drug delivery device can include a single vial that contains enough drug therein for more than two drug sprays. The drug delivery device may therefore be usable to deliver a complete drug dose that requires more than two drug sprays, such as three sprays, four sprays, five sprays, six sprays, etc., which may help improve user familiarity and comfort with the drug delivery device and/or may generate less waste than nasal drug delivery devices that are disposed of after delivering less than all drug sprays therefrom as part of a user's drug dose that requires more than two drug sprays.

The drug to be delivered using a drug delivery device as described herein can be any of a variety of drugs. Examples of drugs that can be delivered using a drug delivery device as described herein include antibodies (such as monoclonal antibodies), hormones, antitoxins, substances for the control of pain, substances for the control of thrombosis, substances for the control of infection, peptides, proteins, human insulin or a human insulin analogue or derivative, polysaccharide, DNA, RNA, enzymes, oligonucleotides, antiallergics, antihistamines, anti-inflammatories, corticosteroids, disease modifying anti-rheumatic drugs, erythropoietin, and vaccines. Examples of drugs that can be delivered using a drug delivery device as described herein include ketamine (e.g., Ketalar^{®}), esketamine (e.g., Spravato^{®}, Ketanest^{®}, and Ketanest-S^{®}), naloxone (e.g., Narcan^{®}), and sumatriptan (e.g., Imitrex^{®}).

A drug delivery device configured to expel a drug into a nose of a patient can have a variety of configurations. In general, the drug delivery device is configured to deliver a drug to a patient, where the drug is provided in a defined dosage form within the drug delivery device.

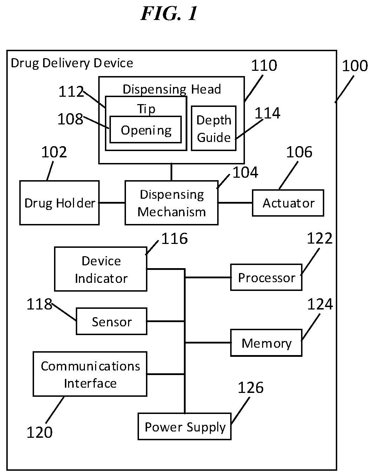

FIG. 1 illustrates one embodiment of a drug delivery device 100 configured to expel a drug into a nose of a patient. As will be appreciated by a person skilled in the art, the drug delivery device 100 can include different features in different embodiments depending upon various requirements, such as the type of drug, typical dosage(s) of the drug, safety requirements in various jurisdictions, whether the device is powered, etc.

The drug delivery device 100 includes a drug holder 102 configured to contain a drug therein for delivery from the device 100 to a patient. The drug holder 102 can have a variety of configurations. In an exemplary embodiment, the drug holder 102 is a vial. An exemplary vial is formed of one or more materials, e.g., glass, polymer(s), etc. In some embodiments, a vial can be formed of glass. In other embodiments, a vial can be formed of one or more polymers. In yet other embodiments, different portions of a vial can be formed of different materials. As discussed further below, the drug holder 102 of the drug delivery device 100 can include a single drug holder 102 or can include a plurality of drug holders 102.

The drug delivery device 100 may not need to be primed after the drug holder 102 has been coupled thereto and prior to actuation of the drug delivery device 100 to cause drug delivery, which may ease use of the drug delivery device 100 by a user.

The drug delivery device 100 also includes a dispensing mechanism 104 that is operatively coupled to the drug holder 102 and configured to drive the drug out of device 100 from the drug holder 102. The dispensing mechanism 104 can have a variety of configurations. For example, the dispensing mechanism 104 can include a plunger configured to push the drug out of the drug holder 102. For another example, the dispensing mechanism 104 can include a piston, pin, and/or a needle configured to pierce through or puncture a seal member of the drug holder 102 in embodiments in which the drug holder 102 includes a pierceable or puncturable seal member.

The drug delivery device 100 also includes an actuator 106 configured to be actuated by a user to cause the dispensing mechanism 104 to begin delivering a dose of the drug through an opening or nozzle 108 in the drug delivery device 100. In an exemplary embodiment, the drug delivery device 100 is configured to be self-administered such that the user who actuates the actuator 106 is the patient receiving the drug from the drug delivery device 100. The actuator 106 can have a variety of configurations, as discussed further below. For example, the actuator 106 can include a plunger. For another example, the actuator 106 can include a pressable button. For still another example, the actuator 106 can include a movable switch. For yet another example, the actuator 106 can include a squeezable body of the drug holder 102.

The opening 108 through which the drug exits the drug delivery device 100 is formed in a dispensing head 110 of the drug delivery device 100 in a tip 112 of the dispensing head 110. The tip 112 is configured to be inserted into a nostril of a patient. In an exemplary embodiment, the tip 112 is configured to be inserted into a first nostril of the patient during a first stage of operation of the drug delivery device 100 and into a second nostril of the patient during a second stage of operation of the drug delivery device 100. The first and second stages of operation involve two separate actuations of the actuator 106, a first actuation corresponding to a first dose of the drug being delivered and a second actuation corresponding to a second dose of the drug being delivered. The dispensing head 110 includes a depth guide 114 configured to contact skin of the patient, e.g., between the patient's first and second nostrils, such that a longitudinal axis of the dispensing head 110 is substantially aligned with a longitudinal axis of the nostril in which the tip 112 is inserted. A person skilled in the art will appreciate that the longitudinal axes may not be precisely aligned but nevertheless be considered to be substantially aligned due to any number of factors, such as manufacturing tolerances and sensitivity of measurement equipment.

In an exemplary embodiment, the dispensing head 110 has a tapered shape in which the dispensing head 110 has a smaller diameter at its distal end than at its proximal end where the opening 108 is located. The opening 108 having a relatively small diameter facilitates spray of the drug out of the opening 108, as will be appreciated by a person skilled in the art. A spray chamber through which the drug is configured to pass before exiting the opening 108 is located within a proximal portion of the tapered dispensing head 110, distal to the opening 108. When the drug passes through the spray chamber at speed, the spray chamber facilitates production of a fine mist that exits through the opening 108 with a consistent spray pattern.

In some embodiments, the dispensing head 110 can include two tips 112 each having an opening 108 therein such that the drug delivery device 100 is configured to simultaneously deliver doses of drug into two nostrils in response to a single actuation of the actuator 106.

The drug delivery device 100 also includes a device indicator 116 configured to present information to a user about a status of the drug delivery device 100 and/or the drug contained in the drug holder 102. The device indicator 116 can be a visual indicator, such as a display screen, one or more lights, one or more colored and/or numbered markings, etc. Alternatively or in addition, the device indicator 116 can be an audio indicator configured to provide sound.

The drug delivery device 100 also includes a sensor 118 configured to sense information relating to the drug delivery device 100 and/or the drug contained in the drug holder 102. Examples of information that the sensor 118 can sense include environmental conditions (e.g., temperature, humidity, geographic location, time, etc.). The drug delivery device 100 can also include a communications interface 120 configured to communicate externally data which has been gathered by the sensor 118 about the drug delivery device 100 and/or the drug contained in the drug holder 102, which may facilitate analysis regarding the patient's treatment, patient compliance, use of the drug delivery device 100, etc.

In embodiments in which the drug delivery device 100 includes one or more electrical components, e.g., the device indicator 116 (which in some embodiments can be powered while in other embodiments not be powered), the sensor 118, the communications interface 120, a processor 122, a memory 124, etc., the drug delivery device 100 includes a power supply 126 configured to deliver electrical power to the one or more electrical components of the drug delivery device 100. The power supply 126 can be a source of power which is integral to drug delivery device 100 and/or can be a mechanism configured to connect the drug delivery device 100 to an external source of power. The processor 122 is configured to receive gathered data from the sensor 118 and to cause the data to be stored in the memory 124, to be indicated on the device indicator 110, and/or to be communicated externally via the communications interface 120. The memory 124 is configured to store instructions that are configured to be executed by the processor 122 for the processor 122 to process information regarding the various electrical components with which the processor 122 is in communication.

As mentioned above, the drug delivery device 100 can include different features in different embodiments depending upon various requirements. For example, the drug delivery device 100 can omit any one or more of the depth guide 114, the device indicator 116, the sensor 118, the communications interface 120, the processor 122, the memory 124, and the power supply 126.

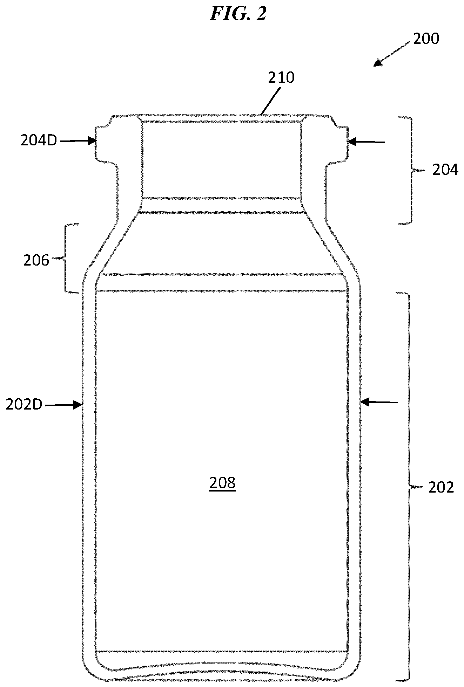

FIG. 2 illustrates an exemplary embodiment of the drug holder 102 in the form of a vial 200 that is configured to contain a drug 202 therein. The vial 200 includes a base or distal portion 202 and a head or proximal portion 204. As shown, the vial 200 also includes an inwardly tapering neck portion 206 that extends between the base portion 202 and the head portion 204. The inwardly tapering neck portion 206 allows the head portion 204 to have a maximum outer diameter 204D that is less than a maximum outer diameter 202D of the base portion 202. In other embodiments, the taper between the base and head portions 202, 204 can be omitted, and the base and head portions 202, 204 can have a same maximum outer diameter 202D, 204D as one another.

The base portion 202 defines a hollow interior 208 within the base portion 202 that is configured to contain the drug therein. In an exemplary embodiment, an amount of the drug in the vial 200 is such that the drug can be contained entirely within the hollow interior 208 of the base portion 202, but the amount of the drug can be such that drug entirely fills the hollow interior 208 of the base portion 202 and also fills at least some part of the neck portion 206 and optionally also at least some part of the head portion 204. While the base portion 202 can have a variety of configurations, in this illustrated embodiment, the base portion 202 has a substantially cylindrical shape. In other embodiments, the base portion 202 can have any other suitable shapes, e.g., a substantially rectangular shape, etc. A person skilled in the art will appreciate that a shape may not be a precise shape (e.g., a precise cylinder or a precise rectangle) but nevertheless be considered to be substantially that shape due to any number of factors, such as manufacturing tolerances and sensitivity of measurement equipment.

The vial 200 includes a seal member 210 at a proximal end thereof in the head portion 204. The seal member 210 is configured to provide a fluid tight seal such that the drug is contained in the vial 200 until the seal provided by the seal member 210 is broken. The seal provided by the seal member 210 can be broken in a variety of ways, such as by being pierced or punctured by a needle of the drug delivery device to which the vial 200 is releasably coupled. The seal member 210 can have a variety of configurations, as will be appreciated by a person skilled in the art, such as by being a pierceable polymer septum or a foil layer. The seal member 210 can be protected from accidental puncturing or piercing before intended use with a removable protective member or stopper, such as a tamper evident (TE) seal, etc. **In** some embodiments, the seal member 210 can be omitted and instead a removable protective member or stopper can be provided that is removed just prior to use of the vial 200.

An exemplary vial can include a variety of features to facilitate sealing and storing a drug therein, as described herein and illustrated in the drawings. However, a person skilled in the art will appreciate that the vials can include only some of these features and/or can include a variety of other features known in the art. The vials described herein are merely intended to represent certain exemplary embodiments.

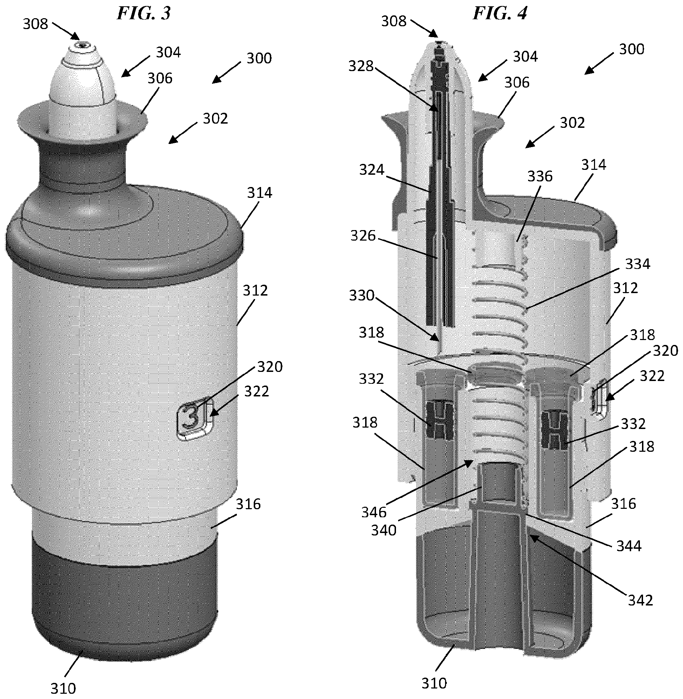

FIGS. 3-8 illustrate an exemplary embodiment of the drug delivery device 100 of FIG. 1. FIGS. 3-8 illustrate a drug delivery device 300 configured to expel a drug into a nose of a patient and that is generally configured and used similar to that discussed above regarding FIG. 1. The drug delivery device 300 includes a dispensing head 302 that includes a tip 304, a depth guide 306, and an opening 308. The tip 304 is configured to be inserted into a nostril to deliver drug into the nostril. The drug delivery device 300 also includes an actuator configured to be actuated to cause drug to exit the drug delivery device 300 through the opening 308. The actuator includes a plunger 310 in this illustrated embodiment. The drug in this illustrated embodiment is configured to be self-administered such that the user who actuates the actuator, e.g., the plunger 310, is the person receiving the drug from the drug delivery device 300, although another person can actuate the device 300 for delivery into another person.

The drug delivery device 300 includes a body 312 with which the tip 304 is integrally formed. In other embodiments, the body 312 can be a separate member from the tip 304 and have the tip 304 attached thereto. The drug delivery device 300 also includes a cap 314 that is a separate member from the body 312, which may facilitate manufacturing of the drug delivery device 300, e.g., by allowing the cap 314 and the body 312 to be separately molded or otherwise formed, by allowing the cap 314 and the body 312 to be made from a different material than one another, etc. The cap 314 is integrally formed with the depth guide 306. In other embodiments, the cap 314 can be a separate member from the depth guide 306, which may facilitate manufacturing of the drug delivery device 300, e.g., by allowing the cap 314 and the depth guide 306 to be separately molded or otherwise formed, by allowing the cap 314 and the depth guide 306 to be made from a different material than one another, etc.

The drug delivery device 300 includes a cartridge 316 configured to seat a plurality of vials 318 therein. The vials 318 are each generally configured and used similar to that discussed above regarding the vial 200 of FIG. 2. The cartridge 316 in this illustrated embodiment is configured to seat six vials 318 therein. In other embodiments, the cartridge 316 can be configured to seat another plural number of vials therein, e.g., two, three, four, five, seven, etc. In this illustrated embodiment, each of the vials 318 contains an amount of drug therein for one spray of the drug to be delivered out of the opening 308. The drug delivery device 300 is thus configured to deliver six drug sprays. In other words, each of the multiple sprays deliverable from the drug delivery device 300 includes drug from only one of the vials 318.

The vials 318 are pre-loaded into the cartridge 316 during manufacturing. The drug delivery device 300 is thus configured to be provided to an end user with the vials 318 already contained therein.

The drug in each of the vials 318 can be the same as one another, or the drug in each of the vials 318 can be different from one or more of the other drugs in the vials 318.

The cartridge 316 includes a plurality of indicators 320 thereon that each correspond to one of the vials 318. The cartridge 316 thus includes six indicators 320 in this illustrated embodiment. The indicators 320 are each configured to uniquely indicate a feature associated with its corresponding one of the vials 318. The indicators 320 in this illustrated embodiment are numbers one, two, three, four, five, and six so as to uniquely identify each of the six vials 318 by number. Instead of or in addition to including a number, the indicators 320 can be color-coded so as to provide unique identification by color, the indicators 320 can be alphabetical so as to provide unique identification by one or more letters, the indicators 320 can be numbers of dots or other symbols so as to provide unique identification by a number of dots or other symbols, or the indicators 320 can have another configuration. The indicators 320 can be provided on the cartridge 316 in any of a variety of ways, such as by being etched therein, molded therein, printed thereon, added thereto via sticker or other type of label, etc.

The indicators 320 are each configured to be visible through a viewing window 322 formed in a sidewall of the body 312. The viewing window 322 is configured to be clearly visible to a user of the drug delivery device 300 when the drug delivery device 300 is held by hand. The viewing window 322 is square-shaped in this illustrated embodiment but can have another shape, e.g., circular, triangular, ovular, rectangular, etc. The drug delivery device 300 includes only one viewing window 322 in this illustrated embodiment but can have another number of viewing windows. For example, a second viewing window can be formed in a side wall of the body 312 substantially 180° around a circumference of the body 312 that is configured and used similar to the viewing window 322. The second viewing window may facilitate a user's visualization of at least one of two viewing windows regardless of whether the user's right or left hand is holding the drug delivery device 300. For another example, a second viewing window configured and used similar to the viewing window 322 can be formed in a side wall of the body 312 either proximal to or distal to the viewing window 322. The second viewing window may facilitate a user's visualization of at least one of two viewing windows regardless of how the user's hand holding the drug delivery device 300 is positioned relative to the body 312 while the user is holding the drug delivery device 300.

The viewing window 322 is configured to cooperate with the indicators 320 to indicate to a user how many drug deliveries remain possible from the drug delivery device 300. The indicators 320 and the viewing window 322 thus also cooperate to inform a user how many drug deliveries have been made from the drug delivery device 300. The indicators 320 are configured as a numerical countdown with the indicator 320 being "6" for the first vial 318 containing drug to be delivered first from the drug delivery device 300 so as to indicate six remaining possible drug deliveries, with the indicator 320 being "5" for the second vial 318 containing drug to be delivered second from the drug delivery device 300 so as to indicate five remaining possible drug deliveries, with the indicator 320 being "4" for the third vial 318 containing drug to be delivered third from the drug delivery device 300 so as to indicate four remaining possible drug deliveries, with the indicator 320 being "3" for the fourth vial 318 containing drug to be delivered fourth from the drug delivery device 300 so as to indicate three remaining possible drug deliveries, with the indicator 320 being "2" for the fifth vial 318 containing drug to be delivered fifth from the drug delivery device 300 so as to indicate two remaining possible drug deliveries, with the indicator 320 being "1" for the sixth vial 318 containing drug to be delivered sixth from the drug delivery device 300 so as to indicate one remaining possible drug delivery, and with the indicator 320 being "0" so as to indicate no remaining possible drug deliveries. FIGS. 3, 4, and 6 illustrate the indicator 320 corresponding to the fourth vial 318, which is the number "3," positioned in the viewing window 322. In other embodiments, the indicators 320 can count up instead of down so as to indicate which dose is to be delivered next from the drug delivery device 300, e.g., with the indicator 320 being "1" for the first vial 318 to indicate that the first spray is to be delivered next, the indicator being "2" for the second vial 318 to indicate that the second spray is to be delivered next, etc. In such embodiments, the viewing window 322 is also configured to cooperate with the indicators 320 to indicate to a user how many drug deliveries remain possible from the drug delivery device 300 and to inform a user how many drug deliveries have been made from the drug delivery device 300.

Some drugs, such as esketamine, ketamine, and other controlled substances, may be required to have drug delivery therefrom verified per the drug's Risk Evaluation and Mitigation Strategies (REMS) to help, e.g., ensure that substantially no drug remains in the drug delivery device so that the drug deliverable therefrom is not accessed by an unauthorized party. The indicators 320 and the viewing window 322 may help provide such verification by indicating whether or not drug from all of the vials 318 has been delivered from the drug delivery device 300.

The drug delivery device 300 includes a cannula 324 and a pin 326 held by the cannula 324. The cannula 324 includes an inner passageway 328 that is in communication with the opening 308 and with an inner passageway 330 of the pin 326. The pin 326 is seated in a distal portion of the inner passageway 328 of the cannula 324 and extends distally from the cannula 324. The pin 326 is configured to pierce or puncture a seal member 332 of each of the vials 318 in succession as each of the vials 318 is sequentially moved proximally relative to the pin 326, as discussed further below. With the pin 326 pierced or punctured through a vial's seal member 332, the drug in the vial 318 can exit the vial 318, then travel proximally in the inner passageway 330 of the pin 326, then travel proximally in the inner passageway 328 of the cannula 324, and then exit the drug delivery device 300 through the opening 308.

The cartridge 316 is configured to rotate relative to the dispensing head 302, the body 312, the cap 314, and the plunger 310. The vials 318 contained in the cartridge 316 are configured to rotate with the cartridge 316 relative to the dispensing head 302, the body 312, the cap 314, and the plunger 310. The rotation of the cartridge 316 allows for each of the vials 318 to be aligned with the tip 304 for delivery of the aligned vial's drug out of the opening 308.

The drug delivery device 300 includes a spring 334 operatively coupled to the plunger 310 and the cartridge 316. The spring 334 is a coil spring in this illustrated embodiment but can be another type of spring or biasing member. The spring 334 is configured to bias the plunger 310 and the cartridge 316 in a distal direction. A proximal end of the spring 334 abuts the body 312, e.g., a proximal spring base 336 of the body 312. A distal end of the spring 334 abuts the plunger 310, e.g., a bottom inner surface 338 of the plunger 310. The spring 334 being fixed at either end thereof to the body 312 and the plunger 310 is configured to allow the plunger 310 to move relative to the body 312.

A proximal extension 340 of the plunger 310 extends through a distal opening 342 of the cartridge 316. The plunger 310 includes a lip 344 that has a diameter greater than a diameter of the distal opening 342. The lip 344 is located in a cavity 346 of the cartridge 316 that is proximal to the distal opening 342. The lip 344 having a diameter greater than a diameter of the distal opening 342 is configured to prevent the proximal extension 340 from moving distally out of the cavity 344 and through the distal opening 342. The cartridge 316 and the plunger 310 are thus attached to one another. Consequently, the spring 334 is also configured to allow the cartridge 316 to move relative to the body 312.

The plunger 310 and the cartridge 316 are each configured to move distally relative to the body 312 and the cap 314 under the biasing force of the spring 334. The distal movement of the cartridge 316 relative to the body 312 is configured to cause the cartridge 316 to rotate relative to the body 312 and relative to the plunger 310.

The cartridge 316 includes a groove 348 formed in an outer surface thereof. The groove 348 includes a plurality of longitudinal grooves 348L and a plurality of transverse grooves 348T. The plurality of longitudinal grooves 348L and a plurality of transverse grooves 348T are in communication with each other to form the continuous groove 348. The plurality of longitudinal grooves 348L and a plurality of transverse grooves 348T alternate with one another along the groove 348. Each of the plurality of longitudinal grooves 348L is substantially parallel to a longitudinal axis of the cartridge 316, which is also the longitudinal axis of the body 312 and the plunger 310. A person skilled in the art will appreciate that the grooves may not be precisely parallel with the longitudinal axes but nevertheless be considered to be substantially parallel thereto due to any number of factors, such as manufacturing tolerances and sensitivity of measurement equipment. Each of the plurality of transverse grooves 348T are at a non-zero, non-right angle relative to the longitudinal grooves 348L and thus to the longitudinal axes of the cartridge 316, the body 312, and the plunger 310. The angle of the plurality of transverse grooves 348T will vary as the angle depends on a particular drug delivery device's configuration, such as a number of vials 318 that the cartridge 316 is configured to seat therein and on a spacing between the vials 318 in the cartridge 318. Each of the longitudinal grooves 348L has a variable depth. Each of the longitudinal grooves 348L becomes shallower in a distal direction.

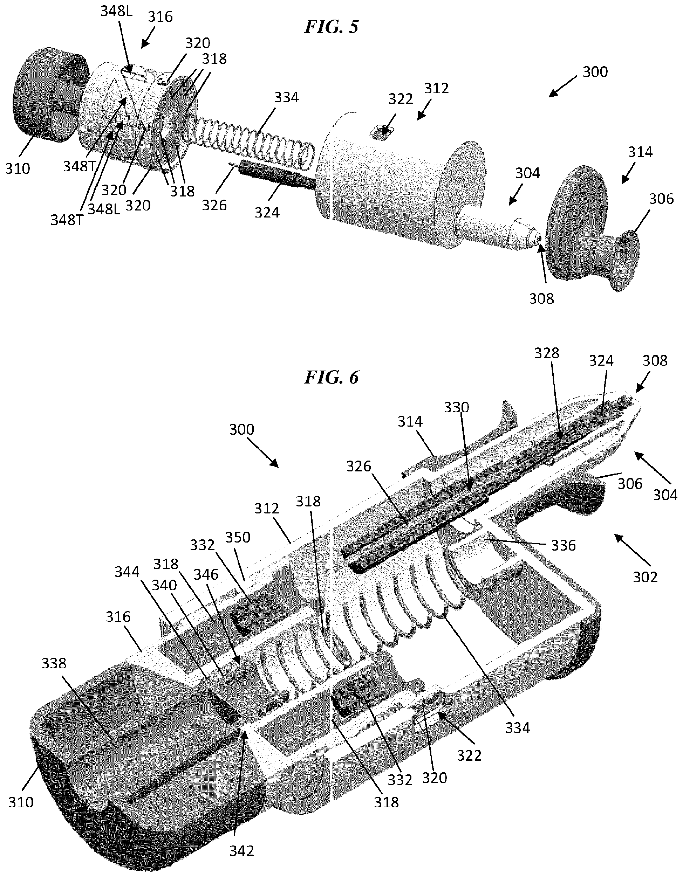

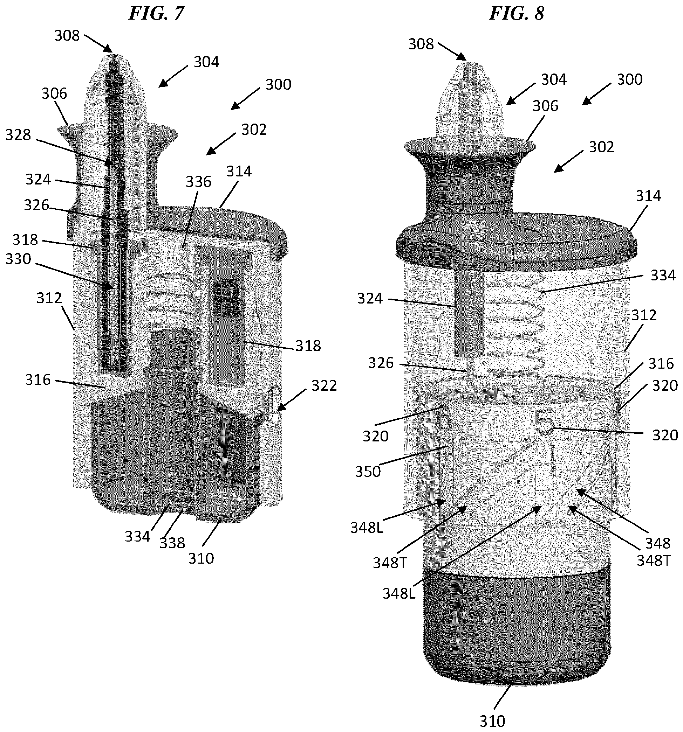

The body 312 includes a protrusion 350 (see FIGS. 6 and 8) that extends radially inward from an inner surface of the body 312. The protrusion 350 is configured to slide in the groove 348 of the cartridge 316. FIG. 8 illustrates the drug delivery device 300 in an initial configuration before a first actuation of the actuator 310. The protrusion 350 in FIG. 8 is thus shown in an initial position relative to the groove 348. The protrusion 350 is positioned at a proximal end of a first one of the longitudinal grooves 348L that is associated with a first one of the vials 318 from which drug will be delivered in response to actuation of the actuator 310. The protrusion 350 has a size and shape complementary to a size and shape of the groove 348 so the protrusion 350 can smoothly slide in the groove 348 and not be disengaged therefrom while the protrusion 350 is sliding in the groove 348. The protrusion 350 is rectangular-shaped in this illustrated embodiment.

The actuation of the plunger 310 includes pushing the plunger 310 in a proximal direction, such as by a user or other element pushing on a distal surface of the plunger 310. The proximal pushing of the plunger 310 causes the plunger 310, and the cartridge 316 engaged therewith, to move proximally relative to the body 312 and the cap 314 and thus relative to the dispensing head 302. With the drug delivery device 300 in this initial configuration, the proximal movement of the cartridge 316 causes the protrusion 350 to ride in the first one of the longitudinal grooves 348L as the cartridge 316 moves proximally. The proximal movement of the cartridge 316 also causes the vials 318 seated therein to move proximally and causes the spring 334 to be compressed. The first one of the vials 318 is aligned with the tip 304 such that the pin 326 pierces or punctures the seal member 332 of the first one of the vials 318, thereby causing the pin's inner passageway 330 to become in fluid communication with the drug in the first one of the vials 318. The drug in the first one of the vials 318 is thus drawn proximally into the pin's inner passageway 326, then into the cannula's inner passageway 328, and then out the opening 308. A spray chamber of the tip 304 defined by the cannula's inner passageway 328 facilitates the drug exiting the opening 308 as a spray, as discussed above. The plunger 310 and the cartridge 316 move proximally under the proximally directed force applied to the plunger 310 until a proximal outer surface of the cartridge 316 abuts a distal inner surface of the body 312, as shown in FIG. 7. The drug has been delivered from the first one of the vials 318. After the first drug has been delivered and the proximal outer surface of the cartridge 316 abuts a distal inner surface of the body 312, the protrusion 350 of the body 312 has moved along the entire length of the first one of the longitudinal grooves 348L of the cartridge 316.

In response to the proximally directed force being removed from the plunger 310, e.g., from the user or other element ceasing to push proximally on the plunger 310, the spring 334 is allowed to expand and thereby cause the plunger 310 and the cartridge 316 to move distally relative to the body 312 and the cap 314 and thus relative to the dispensing head 302. The distal bias of the spring 334 automatically causes the distal movement of the plunger 310 and the cartridge 316. As the cartridge 316 moves distally, the cannula 324 and the pin 326 are moved out of the first one of the vials 318. When the cannula 324 and the pin 326 have moved out of the first one of the vials 318, the cartridge 316 is free to rotate relative to the plunger 310, the body 312, and the cap 314, and thus relative to the dispensing head 302. The variable depth of the longitudinal grooves 348L results in the protrusion 350 being movable in the first one of the transverse grooves 348T instead of in the first one of the longitudinal grooves 350L as the cartridge 316 moves distally after clearing the cannula 324 and the pin 326. The transverse orientation of the first one of the transverse grooves 348T causes the cartridge 316 to rotate as the protrusion 350 slides in the first one of the transverse grooves 348T. The rotation of the cartridge 316, and thus also the vials 318 seated therein, causes the second one of the vials 318 to become aligned with the tip 304 so as to be in position for drug delivery therefrom in response to a second actuation of the plunger 310. The first one of the transverse groove 348T defines how much the cartridge 316 rotates relative to the plunger 310, the body 312, the cap 314, and the dispensing head 302. In this illustrated embodiment, each of the plurality of transverse grooves 348T is such that the cartridge 316 is configured to rotate about 60 degrees as cartridge 316 is also moving distally and the protrusion 350 is sliding in the first one of the transverse grooves 348T. A person skilled in the art will appreciate that a value may not be precisely at a value but nevertheless be considered to be at about that value due to any number of factors, such as manufacturing tolerances and sensitivity of measurement equipment. The protrusion 350 and the groove 348 cooperate to stop the rotation of the cartridge 316. The cartridge 316 stops rotating when the protrusion 350 reaches a proximal end of the first one of the transverse grooves 348T, which positions the protrusion 350 at a proximal end of the second one of the longitudinal grooves 348L. The rotation of the cartridge 316 causes the indicator 320 visible in the viewing window 322 to change.

The plunger 310 can then be actuated a second time to cause drug delivery from the second one of the vials 318 similar to that discussed above regarding the first one of the vials, and so on until drug has been delivered from each of the vials 318.

The drug delivery device 300 in this illustrated embodiment is not powered, e.g., does not include any electrical components such as a processor, a sensor, a memory, a communications interface, etc. The drug delivery device 300 thus does not need to include a power supply though a power supply could be included, e.g., for connectivity, for powering a light source of the drug delivery device, etc.

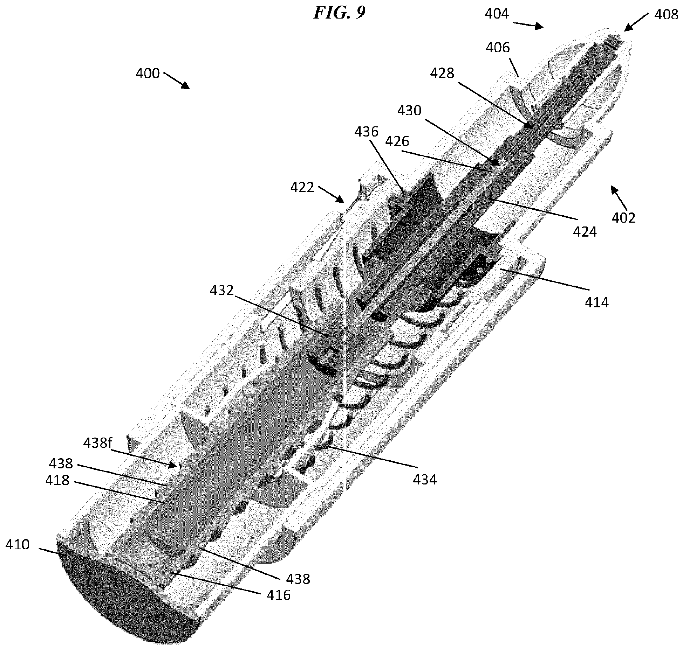

FIGS. 9-12 illustrate another exemplary embodiment of the drug delivery device 100 of FIG. 1. FIGS. 9-12 illustrate a drug delivery device 400 configured to expel a drug into a nose of a patient and that is generally configured and used similar to that discussed above regarding FIG. 1. The drug delivery device 400 includes a single vial 418 that contains enough drug therein for more than two drug sprays. **In** this illustrated embodiment the vial 418 contains enough drug therein for six sprays although another number is possible, e.g., three, four, five, seven, etc. The drug delivery device 400 is thus configured to deliver six drug sprays. **In** other words, each of the multiple sprays deliverable from the drug delivery device 400 includes drug from the one vial 418. The vial 418 is generally configured and used similar to that discussed above regarding the vial 200 of FIG. 2.

**In** this illustrated embodiment, the drug delivery device 400 includes a dispensing head 402 that includes a tip 404, a depth guide 406, and an opening 408. The tip 404 is configured to be inserted into a nostril to deliver drug into the nostril. The drug delivery device 400 also includes an actuator configured to be actuated to cause drug to exit the drug delivery device 400 through the opening 408. The actuator includes a plunger 410 in this illustrated embodiment. The drug in this illustrated embodiment is configured to be self-administered such that the user who actuates the actuator, e.g., the plunger 410, is the person receiving the drug from the drug delivery device 400, although another person can actuate the device 400 for delivery into another person.

The drug delivery device 400 includes a body 412 with which the tip 404 and the depth guide 406 are integrally formed. In other embodiments, the body 412 can be a separate member from the tip 404 and/or the depth guide 406 and have the tip 404 and/or the depth guide 406 attached thereto.

The drug delivery device 400 includes a vial holder 416 configured to seat the vial 418 therein. The vial 418 is pre-loaded into the vial holder 416 during manufacturing. The drug delivery device 400 is thus configured to be provided to an end user with the vial 418 already contained therein.

The drug delivery device 400 includes an indicator collar 414 with a plurality of indicators 420 thereon that each correspond to one of the drug deliveries possible from vial 418. The indicator collar 414 thus includes six indicators 420 in this illustrated embodiment. The indicators 420 are each configured to uniquely indicate a feature associated with its corresponding one of the drug deliveries. The indicators 420 in this illustrated embodiment are numbers one, two, three, four, five, and six so as to uniquely identify each of the drug deliveries by number. Instead of or in addition to including a number, the indicators 420 can be color-coded so as to provide unique identification by color, the indicators 420 can be alphabetical so as to provide unique identification by one or more letters, the indicators 420 can be numbers of dots or other symbols so as to provide unique identification by a number of dots or other symbols, or the indicators 420 can have another configuration. The indicators 420 can be provided on the indicator collar 414 in any of a variety of ways, such as by being etched therein, molded therein, printed thereon, added thereto via sticker or other type of label, etc.

The indicators 420 are each configured to be visible through a viewing window 422 formed in a sidewall of the body 412. The viewing window 422 is configured to be clearly visible to a user of the drug delivery device 400 when the drug delivery device 400 is held by hand. The viewing window 422 is square-shaped in this illustrated embodiment but can have another shape, e.g., circular, triangular, ovular, rectangular, etc. The drug delivery device 400 includes only one viewing window 422 in this illustrated embodiment but can have another number of viewing windows. For example, a second viewing window can be formed in a side wall of the body 412 substantially 180° around a circumference of the body 412 that is configured and used similar to the viewing window 422. The second viewing window may facilitate a user's visualization of at least one of two viewing windows regardless of whether the user's right or left hand is holding the drug delivery device 400. For another example, a second viewing window configured and used similar to the viewing window 422 can be formed in a side wall of the body 412 either proximal to or distal to the viewing window 422. The second viewing window may facilitate a user's visualization of at least one of two viewing windows regardless of how the user's hand holding the drug delivery device 400 is positioned relative to the body 412 while the user is holding the drug delivery device 400.

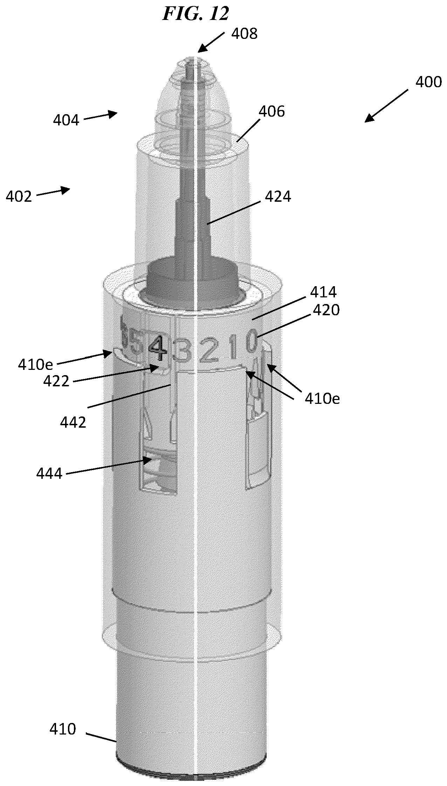

The viewing window 422 is configured to cooperate with the indicators 420 to indicate to a user how many drug deliveries remain possible from the drug delivery device 400. The indicators 420 and the viewing window 422 thus also cooperate to inform a user how many drug deliveries have been made from the drug delivery device 400. The indicators 420 are configured as a numerical countdown with the indicator 420 being "6" for the first drug spray to be delivered from the drug delivery device 400 so as to indicate six remaining possible drug deliveries, with the indicator 420 being "5" for the second drug spray to be delivered second from the drug delivery device 400 so as to indicate five remaining possible drug deliveries, with the indicator 420 being "4" for the third drug spray to be delivered third from the drug delivery device 400 so as to indicate four remaining possible drug deliveries, with the indicator 420 being "3" for the drug spray to be delivered fourth from the drug delivery device 400 so as to indicate three remaining possible drug deliveries, with the indicator 420 being "2" for the fifth to be delivered fifth from the drug delivery device 400 so as to indicate two remaining possible drug deliveries, with the indicator 420 being "1" for the sixth drug spray to be delivered sixth from the drug delivery device 400 so as to indicate one remaining possible drug delivery, and with the indicator 420 being "0" so as to indicate no remaining possible drug deliveries. FIG. 12 illustrates the indicator 420 corresponding to the third drug spray, which is the number "4," positioned in the viewing window 422. In other embodiments, the indicators 420 can count up instead of down so as to indicate which dose is to be delivered next from the drug delivery device 400, e.g., with the indicator 420 being "1" for the first drug spray to indicate that the first spray is to be delivered next, the indicator being "2" for the second drug spray to indicate that the second spray is to be delivered next, etc. In such embodiments, the viewing window 422 is also configured to cooperate with the indicators 420 to indicate to a user how many drug deliveries remain possible from the drug delivery device 400 and to inform a user how many drug deliveries have been made from the drug delivery device 400.

Some drugs, such as controlled substances, may be required to have drug delivery therefrom verified per the drug's REMS to help, e.g., ensure that substantially no drug remains in the drug delivery device so that the drug deliverable therefrom is not accessed by an unauthorized party. The indicators 420 and the viewing window 422 may help provide such verification by indicating whether or not substantially all of the drug in the vial 418 has been delivered from the drug delivery device 400 by indicating whether or not all six drug deliveries have been made. A person skilled in the art will appreciate that 100% of the drug may not have exited the vial 418, but the vial 418 can nevertheless be considered to have substantially no drug therein due to any number of factors, such as sensitivity of measurement equipment.

The drug delivery device 400 includes a cannula 424 and a pin 426 held by the cannula 424. The cannula 424 includes an inner passageway 428 that is in communication with the opening 408 and with an inner passageway 430 of the pin 426. The pin 426 is seated in a distal portion of the inner passageway 428 of the cannula 424 and extends distally from the cannula 424. The pin 426 is configured to pierce or puncture a seal member 432 of the vial 418, as discussed further below. With the pin 426 pierced or punctured through the vial's seal member 432, the drug in the vial 418 can exit the vial 418, then travel proximally in the inner passageway 430 of the pin 426, then travel proximally in the inner passageway 428 of the cannula 424, and then exit the drug delivery device 400 through the opening 408.

The vial holder 416 is configured to move longitudinally relative to the body 412 and relative to the dispensing head 402. The vial 418 is configured to move longitudinally with the vial holder 416 relative to the dispensing head 402 and the body 412. The longitudinal movement of the vial holder 416 allows for the vial 418 to be positioned relative to the plunger 410 and the body 412 for delivery of the next drug spray out of the opening 408. In other words, the longitudinal movement of the vial holder 416 allows for the vial 418 to be positioned at a location relative to the body 412 such that actuation of the plunger 410 causes the next drug spray to be delivered out of the opening 408 up to and including the maximum number of possible drug sprays, which is six sprays in this illustrated embodiment.

The drug delivery device 400 includes a spring 434 operatively coupled to the plunger 410. The spring 434 is a coil spring in this illustrated embodiment but can be another type of spring or biasing member. The spring 434 is configured to bias the plunger 410 in a distal direction. A proximal end of the spring 434 abuts a base collar 436, e.g., a distal surface thereof. The base collar 436 is a separate member from the body 412 and is fixedly attached to the body 412. The base collar 436 being a separate member from the body 412 may facilitate manufacturing of the drug delivery device 400, e.g., by allowing the base collar 436 and the body 412 to be separately molded or otherwise formed, by allowing the base collar 436 and the body 412 to be made from a different material than one another, etc. In other embodiments, the body 412 can be integrally formed with the base collar 436. A distal end of the spring 434 abuts the plunger 410, e.g., a proximal surface thereof. The spring 434 being fixed at either end thereof to the base collar 436 (and hence the body 412 in fixed relation therewith) and the plunger 410 is configured to allow the plunger 410 to move relative to the body 412.

The plunger 410 is configured to move distally relative to the body 412, the dispensing head 402, the vial holder 416, and the vial 418 under the biasing force of the spring 434. The distal movement of the plunger 410 relative to the vial holder 416 is configured to facilitate positioning of the vial holder 416, and thus also the vial 418, to be positioned at a location relative to the body 412 such that a next actuation of the plunger 410 causes the next drug spray to be delivered out of the opening 408 up to and including the maximum number of possible drug sprays.

The vial holder 416 includes a plurality of teeth 438 formed in an outer surface thereof. As in this illustrated embodiment, a number of the teeth 438 can be one more than the maximum number of possible drug sprays from the drug delivery device 400. The drug delivery device 400 thus includes seven teeth 438 in this illustrated embodiment. Each of the teeth 438 has a distal face 438f configured to sequentially engage the plunger 410. The teeth 438 each extend circumferentially around the vial holder 416. The distal face 438f of each of the teeth 438f is thus ring-shaped. The teeth 438 extending circumferentially around the vial holder 416 may facilitate engagement of the teeth 438 with the plunger 410 regardless of a rotational orientation at which the vial holder 416 is positioned relative to the plunger 410. In other embodiments, the teeth 438 can each include one or more arcuate extensions so as to form an arc (one extension) or a broken ring (a plurality of extensions) around the perimeter of the vial holder 416.

The plunger 410 includes a proximal surface 440 configured to sequentially engage the teeth 438 in a proximal-to-distal direction. FIG. 9 illustrates the drug delivery device 400 in an initial configuration before a first actuation of the actuator 410. FIG. 9 illustrates the proximal surface 440 engaged with a distal face 438f of the first one of the teeth 438, which is the proximal-most one of the teeth 438. The plunger 410 and the vial holder 416 (and the vial 418 held thereby) in FIG. 9 are thus shown in an initial position relative to one another.

The actuation of the plunger 410 includes pushing the plunger 410 in a proximal direction, such as by a user or other element pushing on a distal surface of the plunger 410. A length of the plunger's proximal movement corresponds to one dose of drug being delivered from the drug delivery device 400. The proximal pushing of the plunger 410 causes the plunger 410, and the vial holder 416 engaged therewith, to move proximally relative to the body 412 and thus relative to the dispensing head 402. The vial 418 held by the vial holder 416 also moves proximally relative to the body 412 and the dispensing head 402. With the drug delivery device 400 in this initial configuration, the proximal pushing of the plunger 410 causes the plunger 410, the vial 418, and the vial holder 416 engaged with the plunger 410 via the engaged proximal surface 440 and the distal face 438f of the first one of the teeth 438, to move proximally relative to the body 412 and the dispensing head 402. The proximal movement of the plunger 410 causes the spring 434 to be compressed. The vial 418 is aligned with the tip 404 such that the pin 426 pierces or punctures the seal member 432 of the vial 418, thereby causing the pin's inner passageway 430 to become in fluid communication with the drug in the vial 418. The drug in the vial 418 is thus drawn proximally into the pin's inner passageway 426, then into the cannula's inner passageway 428, and then out the opening 408. A spray chamber of the tip 404 defined by the cannula's inner passageway 428 facilitates the drug exiting the opening 408 as a spray, as discussed above. The drug has been delivered from the vial 418 in a first spray. The body 412 includes a plurality of ribs 442 (see FIG. 12) that extend radially outward therefrom that each engage a corresponding channel 444 formed in a proximal portion of the plunger 410. The engagement of the ribs 442 and the channels 444 prevent the plunger 410 from rotating relative to the body 412 while allowing the plunger 410 to translate longitudinally relative to the body 412 in the proximal and distal directions.

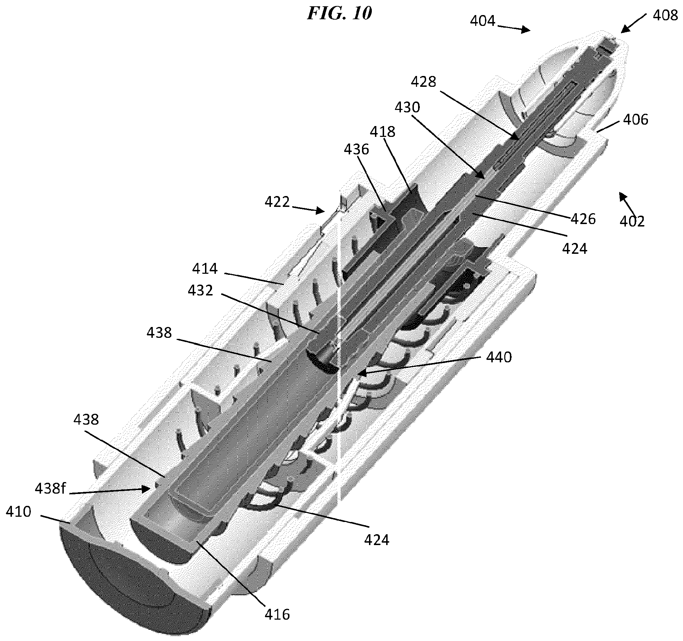

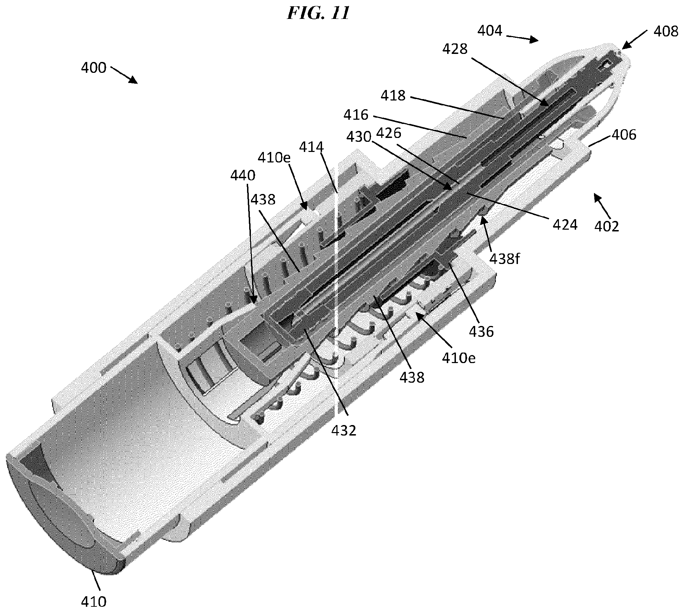

In response to the proximally directed force being removed from the plunger 410, e.g., from the user or other element ceasing to push proximally on the plunger 410, the spring 434 is allowed to expand and thereby cause the plunger 410 to move distally relative to the body 412, the dispensing head 402, the vial holder 416, and the vial 418. The distal bias of the spring 434 automatically causes the distal movement of the plunger 410. As the plunger 410 moves distally, the proximal surface 440 of the plunger 410 moves out of engagement with the distal face 438f of the first one of the teeth 438 and slides along an outer surface 438s of the second one of the teeth 438 that is the second-most proximal one of the teeth 438. The outer surface 438s of each of the teeth 438 is sloped radially outward. A proximal portion 410p of the plunger 410 that includes the proximal surface 440 flexes radially outward in response to the plunger 410 sliding distally along the tooth's sloped surface 438s. When the proximal surface 440 passes just distally past the second one of the teeth 438, the proximal portion 410 flexes radially inward such that the proximal surface 440 of the plunger 410 engages the distal face 438f of the second one of the teeth 438, as shown in FIG. 10. The teeth 438 and the plunger 410 thus act as a ratchet mechanism. The plunger 410 can then be actuated a second time to cause a second spray of drug from the vial 418 similar to that discussed above regarding the first spray, and so on until the maximum number of drug sprays has been delivered. As mentioned above, a length of the plunger's proximal movement in each actuation corresponds to one dose of drug being delivered from the drug delivery device 400. After the final, e.g., sixth, actuation, the proximal surface 440 is engaged with the distal face 438f of the seventh tooth 438, which is the distal-most one of the teeth 438, as shown in FIG. 11.

The indicator collar 414 is operatively engaged with the plunger 410 such that each time the plunger 410 moves distally under force of the spring 434, the indicator collar 414 rotates to cause the next sequential indicator 420 to be visible in the viewing window 422. FIGS. 11 and 12 illustrate the operative engagement of the indicator collar 414 and the plunger 410 with proximal extensions 410e of the plunger 410 engaged with the indicator collar 414.

The drug delivery device 400 in this illustrated embodiment is not powered, e.g., does not include any electrical components such as a processor, a sensor, a memory, a communications interface, etc. The drug delivery device 400 thus does not need to include a power supply though a power supply could be included, e.g., for connectivity, for powering a light source of the drug delivery device, etc.

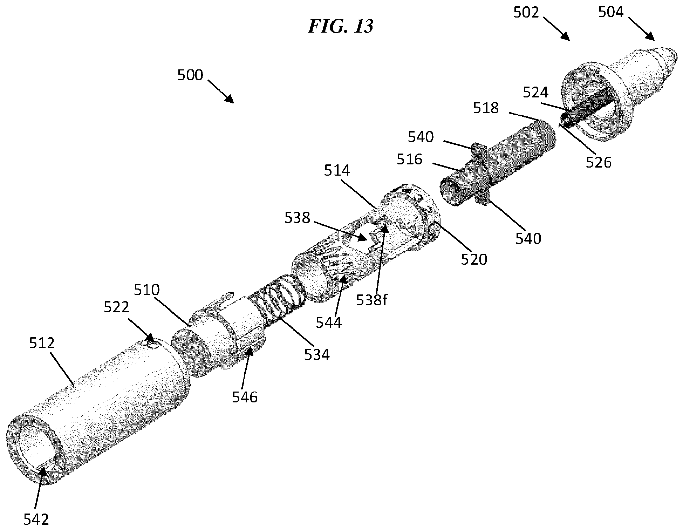

FIGS. 13-17 illustrate another exemplary embodiment of the drug delivery device 100 of FIG. 1. FIGS. 13-17 illustrate a drug delivery device 500 configured to expel a drug into a nose of a patient. The drug delivery device 500 is generally configured and used similar to the drug delivery device 400 of FIGS. 8-12 except that the drug delivery device 500 includes a different ratchet mechanism.

The drug delivery device 500 includes a single vial 518 that contains enough drug therein for more than two drug sprays. In this illustrated embodiment the vial 518 contains enough drug therein for six sprays although another number is possible, e.g., three, four, five, seven, etc. The drug delivery device 500 is thus configured to deliver six drug sprays. In other words, each of the multiple sprays deliverable from the drug delivery device 500 includes drug from the one vial 518. The vial 518 is generally configured and used similar to that discussed above regarding the vial 200 of FIG. 2.

In this illustrated embodiment, the drug delivery device 500 includes a dispensing head 502 that includes a tip 504, a depth guide 506, and an opening 508. The tip 504 is configured to be inserted into a nostril to deliver drug into the nostril. The drug delivery device 500 also includes an actuator configured to be actuated to cause drug to exit the drug delivery device 500 through the opening 508. The actuator includes a plunger 510 in this illustrated embodiment. The drug in this illustrated embodiment is configured to be self-administered such that the user who actuates the actuator, e.g., the plunger 510, is the person receiving the drug from the drug delivery device 500, although another person can actuate the device 500 for delivery into another person.

The drug delivery device 500 includes a body 512 that is a separate member from the dispensing head 502. In other embodiments, the body 512 can be integrally formed with a portion of the dispensing head 502, the tip 504 and/or depth guide 506 thereof, and/or with the entire dispensing head 502.

The drug delivery device 500 includes a vial holder 516 configured to seat the vial 518 therein. The vial 518 is pre-loaded into the vial holder 516 during manufacturing. The drug delivery device 500 is thus configured to be provided to an end user with the vial 518 already contained therein.

The drug delivery device 500 includes a collar 514 with a plurality of indicators 520 thereon that each correspond to one of the drug deliveries possible from vial 518. The collar 514 thus includes six indicators 520 in this illustrated embodiment. The indicators 520 are each configured to uniquely indicate a feature associated with its corresponding one of the drug deliveries. The indicators 520 in this illustrated embodiment are numbers one, two, three, four, five, and six so as to uniquely identify each of the drug deliveries by number. Instead of or in addition to including a number, the indicators 520 can be color-coded so as to provide unique identification by color, the indicators 520 can be alphabetical so as to provide unique identification by one or more letters, the indicators 520 can be numbers of dots or other symbols so as to provide unique identification by a number of dots or other symbols, or the indicators 520 can have another configuration. The indicators 520 can be provided on the collar 514 in any of a variety of ways, such as by being etched therein, molded therein, printed thereon, added thereto via sticker or other type of label, etc.

The indicators 520 are each configured to be visible through a viewing window 522 formed in a sidewall of the body 512. The viewing window 522 is configured to be clearly visible to a user of the drug delivery device 500 when the drug delivery device 500 is held by hand. The viewing window 522 is square-shaped in this illustrated embodiment but can have another shape, e.g., circular, triangular, ovular, rectangular, etc. The drug delivery device 500 includes only one viewing window 522 in this illustrated embodiment but can have another number of viewing windows. For example, a second viewing window can be formed in a side wall of the body 512 substantially 180° around a circumference of the body 512 that is configured and used similar to the viewing window 522. The second viewing window may facilitate a user's visualization of at least one of two viewing windows regardless of whether the user's right or left hand is holding the drug delivery device 500. For another example, a second viewing window configured and used similar to the viewing window 522 can be formed in a side wall of the body 512 either proximal to or distal to the viewing window 522. The second viewing window may facilitate a user's visualization of at least one of two viewing windows regardless of how the user's hand holding the drug delivery device 500 is positioned relative to the body 512 while the user is holding the drug delivery device 500.

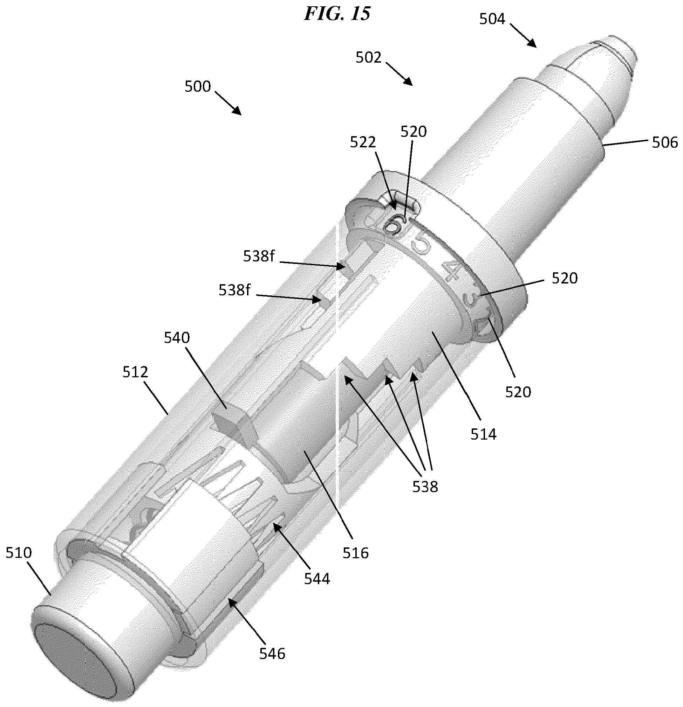

The viewing window 522 is configured to cooperate with the indicators 520 to indicate to a user how many drug deliveries remain possible from the drug delivery device 500. The indicators 520 and the viewing window 522 thus also cooperate to inform a user how many drug deliveries have been made from the drug delivery device 500. The indicators 520 are configured as a numerical countdown with the indicator 520 being "6" for the first drug spray to be delivered from the drug delivery device 500 so as to indicate six remaining possible drug deliveries, with the indicator 520 being "5" for the second drug spray to be delivered second from the drug delivery device 500 so as to indicate five remaining possible drug deliveries, with the indicator 520 being "4" for the third drug spray to be delivered third from the drug delivery device 500 so as to indicate four remaining possible drug deliveries, with the indicator 520 being "3" for the drug spray to be delivered fourth from the drug delivery device 500 so as to indicate three remaining possible drug deliveries, with the indicator 520 being "2" for the fifth to be delivered fifth from the drug delivery device 500 so as to indicate two remaining possible drug deliveries, with the indicator 520 being "1" for the sixth drug spray to be delivered sixth from the drug delivery device 500 so as to indicate one remaining possible drug delivery, and with the indicator 520 being "0" so as to indicate no remaining possible drug deliveries. FIG. 15 illustrates the indicator 520 corresponding to the first drug spray, which is the number "6," positioned in the viewing window 522. In other embodiments, the indicators 520 can count up instead of down so as to indicate which dose is to be delivered next from the drug delivery device 500, e.g., with the indicator 520 being "1" for the first drug spray to indicate that the first spray is to be delivered next, the indicator being "2" for the second drug spray to indicate that the second spray is to be delivered next, etc. In such embodiments, the viewing window 522 is also configured to cooperate with the indicators 520 to indicate to a user how many drug deliveries remain possible from the drug delivery device 500 and to inform a user how many drug deliveries have been made from the drug delivery device 500.

Some drugs, such as esketamine, ketamine, and other controlled substances, may be required to have drug delivery therefrom verified per the drug's REMS to help, e.g., ensure that substantially no drug remains in the drug delivery device so that the drug deliverable therefrom is not accessed by an unauthorized party. The indicators 520 and the viewing window 522 may help provide such verification by indicating whether or not substantially all of the drug in the vial 518 has been delivered from the drug delivery device 500 by indicating whether or not all six drug deliveries have been made. A person skilled in the art will appreciate that 100% of the drug may not have exited the vial 518, but the vial 518 can nevertheless be considered to have substantially no drug therein due to any number of factors, such as sensitivity of measurement equipment.

The drug delivery device 500 includes a cannula 524 and a pin 526 held by the cannula 525. The cannula 524 includes an inner passageway 528 that is in communication with the opening 508 and with an inner passageway 530 of the pin 526. The pin 526 is seated in a distal portion of the inner passageway 528 of the cannula 524 and extends distally from the cannula 525. The pin 526 is configured to pierce or puncture a seal member 532 of the vial 518, as discussed further below. With the pin 526 pierced or punctured through the vial's seal member 532, the drug in the vial 518 can exit the vial 518, then travel proximally in the inner passageway 530 of the pin 526, then travel proximally in the inner passageway 528 of the cannula 524, and then exit the drug delivery device 500 through the opening 508.

The vial holder 516 is configured to move longitudinally relative to the body 512 and relative to the dispensing head 502. The vial 518 is configured to move longitudinally with the vial holder 516 relative to the dispensing head 502 and the body 512. The longitudinal movement of the vial holder 516 allows for the vial 518 to be positioned relative to the plunger 510 and the body 512 for delivery of the next drug spray out of the opening 508. **In** other words, the longitudinal movement of the vial holder 516 allows for the vial 518 to be positioned at a location relative to the body 512 such that actuation of the plunger 510 causes the next drug spray to be delivered out of the opening 508 up to and including the maximum number of possible drug sprays, which is six sprays in this illustrated embodiment.

The drug delivery device 500 includes a spring 534 operatively coupled to the vial holder 516. The spring 534 is a coil spring in this illustrated embodiment but can be another type of spring or biasing member. The spring 534 is configured to bias the vial holder 516 in a proximal direction. A proximal end of the spring 534 abuts the vial holder 516, e.g., a distal surface thereof. A distal end of the spring 534 abuts the plunger 510, e.g., a bottom inner surface thereof. The spring 534 being fixed at either end thereof to the vial holder 516 and the plunger 510 is configured to allow the vial holder 516 to move relative to the body 512, the collar 514, the plunger 510, the cannula 524, the pin 526, and the dispensing head 502.

The vial holder 516 is configured to move proximally relative to the body 512, the collar 514, the plunger 510, the cannula 524, the pin 526, and the dispensing head 502 under the biasing force of the spring 534. The proximal movement of cannula 524, the pin 526, and the dispensing head 502 is configured to facilitate spraying of the drug from the vial 518 and out of the opening 508 and to facilitate positioning of the vial holder 516, and thus also the vial 518, at a location relative to the body 512, the collar 514, the plunger 510, the cannula 524, the pin 526, and the dispensing head 502 such that a next actuation of the plunger 510 causes the next drug spray to be delivered out of the opening 508 up to and including the maximum number of possible drug sprays.

The collar 514 includes a plurality of ratchet steps 538 formed in an outer surface thereof. Two pairs of ratchet steps 538 are formed on opposed sides of the collar 514, as shown in FIG. 13. The pairs of ratchet steps 538 are located about 180° degrees from each other around the collar's circumference. A number of the ratchet steps 538 in each pair is one more than the maximum number of possible drug sprays from the drug delivery device 500. The drug delivery device 500 thus includes seven ratchet steps 538 in this illustrated embodiment in each pair of ratchet steps 538 for a total of fourteen ratchet steps 538. Each of the ratchet steps 538 has a distal face 538f configured to sequentially engage a tab 540 of the vial holder 516, e.g., a proximal surface 540s of the tab 540. The vial holder 516 includes a pair of tabs 540. Each of the tabs 540 is configured to engage one of the pairs of ratchet steps 538. The tabs 540 thus extend from opposed sides of the vial holder 516 and are located about 180° degrees from each other around the vial holder's circumference. Each of the tabs 540 is configured to sequentially engage its associated pair of ratchet steps 538 in a distal-to-proximal direction. The tabs 540 each have a rectangular shape in this illustrated embodiment but can have another shape, e.g., square, trapezoidal, pyramidal, etc.

The body 512 includes longitudinal grooves 542 in which the flange 540 is seated. The flange 540 is configured to slide in the grooves 542. The engagement of the grooves 542 and the flange 540 prevents the vial holder 516 from rotating relative to the body 512 while allowing the vial holder 516 to translate longitudinally relative to the body 512 in the proximal direction.

The collar 514 also includes a track 544 formed therein. The track 544 is located distal to the ratchet steps 538. The track 544 has a zig-zag shape. The plunger 510 includes a protrusion that protrudes radially inward from an inner surface of the plunger 510 and that is configured to slide in the track 544. The track 544 and the protrusion cooperate to allow rotation of the collar 514 relative to the plunger 510, the body 512, the dispensing head 502, the vial holder 516, and the vial 518. The rotation of the collar 514 facilitates the tabs 540 being engaged with different ones of the ratchet steps 538. The rotation of the collar 514 also allows for different ones of the indicators 520 to be visible in the viewing window 522.

The body 512 includes longitudinal ribs configured to be seated in longitudinal channels 546 of the plunger 510. The drug delivery device 500 includes two longitudinal ribs and two longitudinal channels 546 but can include another equal number of each. The engagement of the longitudinal ribs and the longitudinal channels 546 prevents the plunger 510 from rotating relative to the body 512 while allowing the plunger 510 to translate longitudinally relative to the body 512 in the proximal and distal directions.

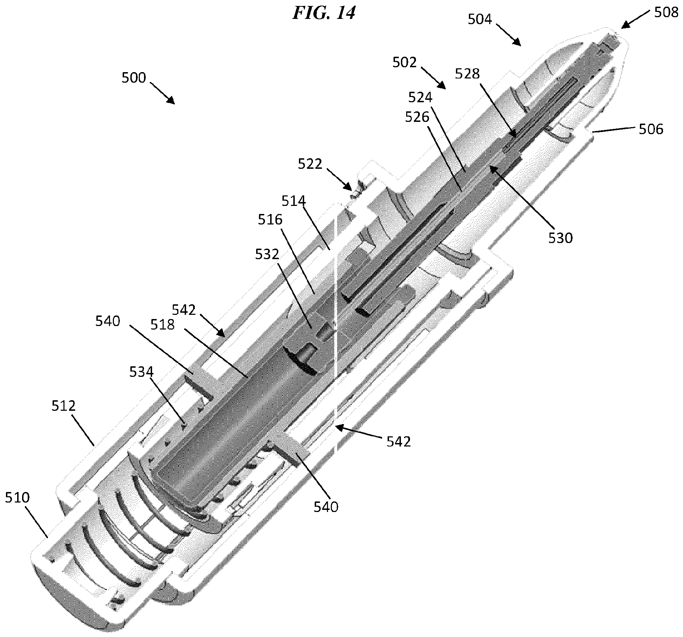

FIGS. 14 and 15 illustrates the drug delivery device 500 in an initial configuration before a first actuation of the actuator 510. FIGS. 14 and 15 illustrate each of the tabs 540 engaged with the distal face 538f of a first one of the ratchet steps 538, which is the distal-most one of the ratchet steps 538, in the tab's associated pair of ratchet steps 538. The plunger 510 and the vial holder 516 (and the vial 518 held thereby) in FIGS. 14 and 15 are thus shown in an initial position relative to one another and relative to the collar 514.

The actuation of the plunger 510 includes pushing the plunger 510 in a proximal direction, such as by a user or other element pushing on a distal surface of the plunger 510. The proximal pushing of the plunger 510 causes the plunger 510 to move proximally relative to the body 512, the vial holder 516, the vial 518, and the dispensing head 502. The plunger's protrusion slides proximally in the collar's track 544 as the plunger 510 moves proximally. The track 544 extending transversely relative to a longitudinal axis of the collar 514, which is also a longitudinal axis of the drug delivery device 500, causes the collar 514 to rotate relative to the vial holder 516, and the vial 518 held thereby, as the plunger 510 moves proximally. As the collar 514 rotates, the tabs 540 of the vial holder 516 each slide along the distal face 538f of the one of the ratchet steps 538 in the tab's associated pair of ratchet steps 538 which with the tab 540 is engaged. The protrusion of the plunger 510 is at a top or peak of one of the zig-zags of the track 544 while the tabs 540 are each sliding along the one of the ratchet steps 538. The collar 514 eventually rotates enough that the tabs 538 become disengaged with the distal face 538f of the one of the ratchet steps 538 in the tab's associated pair of ratchet steps 538. The vial holder 516 now being free of ratchet steps 538 allows the spring 534 to expand and thereby cause the vial holder 516, and the vial 518 held by the vial holder 516, to move proximally relative to the body 512, the collar 514, the plunger 510, the cannula 524, the pin 526, and the dispensing head 502. The vial holder 516 and the vial 518 stop moving proximally when the tabs 540 engage a next-most proximal one of the ratchet steps 538 and engage the distal face 538f thereof. The ratchet steps 538 of the collar 514 and the tabs 540 of the vial holder 516 thus act as a ratchet mechanism. The proximal movement of the vial holder 516 causes the protrusion of the plunger 510 to slide in the track 544 until the protrusion is located at a bottom of a next one of the zig-zags of the track 544.

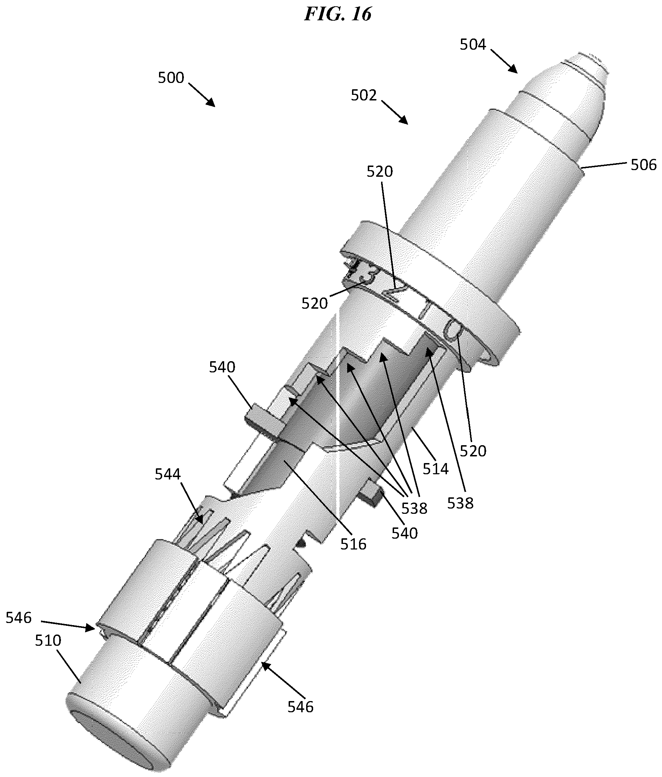

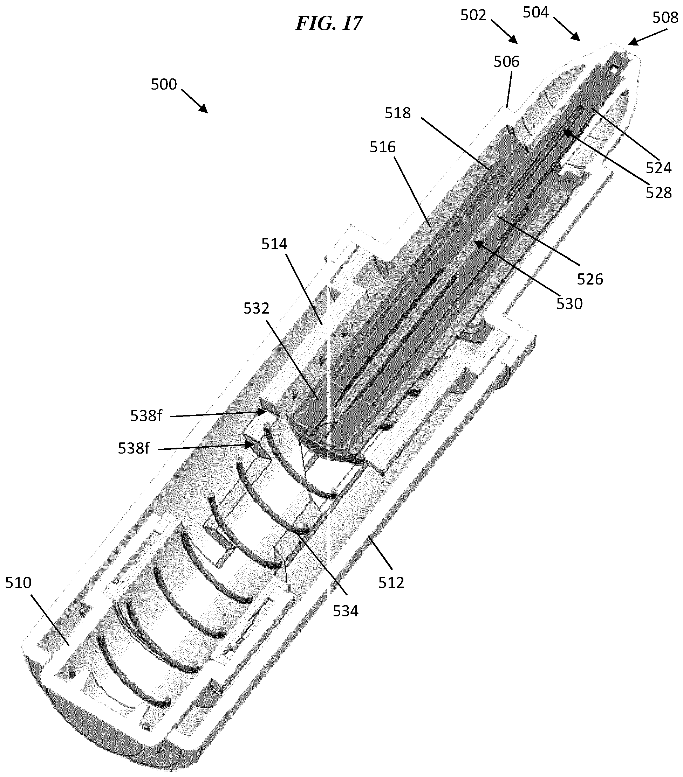

With the drug delivery device 500 in the initial configuration shown in FIGS. 14 and 15, the vial 518 is aligned with the tip 504 such that the proximal pushing of the plunger 510 causes the pin 526 to pierce or puncture the seal member 532 of the vial 518, thereby causing the pin's inner passageway 530 to become in fluid communication with the drug in the vial 518. The drug in the vial 518 is thus drawn proximally into the pin's inner passageway 526, then into the cannula's inner passageway 528, and then out the opening 508. A spray chamber of the tip 504 defined by the cannula's inner passageway 528 facilitates the drug exiting the opening 508 as a spray, as discussed above. The drug has been delivered from the vial 518 in a first spray. The tabs 540 are seated in a second, next-most proximal one of the ratchet steps 538, as shown in FIG. 16. The rotation of the collar 514 has caused a next one of the indicators 520 to be visible in the viewing window 522. The plunger 510 can then be actuated a second time to cause a second spray of drug from the vial 518 similar to that discussed above regarding the first spray, and so on until the maximum number of drug sprays has been delivered. After the final, e.g., sixth, actuation, shown in FIG. 17, the tabs 540 are each engaged with the distal face 538f of the seventh, proximal-most one of the ratchet steps 538.

As shown in FIGS. 13 and 15, a distance between the first, distal-most ratchet step 538 and the second, next-distal-most ratchet step 538 is greater than a distance between each subsequent ratchet step 538. The larger distance takes into account that in the first actuation, the vial holder 516 and the vial 518 need to move enough proximally for the pin 526 to pierce or puncture the seal member 532 and take up any air bubble distal to the seal member 532 before the pin 526 encounters the drug in the vial 518. For each subsequent actuation, the seal member 534 is already pierced or punctured by the pin 526, so the distance is shorter.

The amount that the collar 514 rotates in each actuation varies depending on a configuration of the drug delivery device 500, e.g., on a maximum number of drug sprays possible from the drug delivery device 500, on a size and shape of the track 544, etc. **In** this illustrated embodiment, the collar 514 is configured to rotate about twenty-two degrees in each actuation.

The distance traveled proximally by the plunger 510 in each actuation varies depending on a configuration of the drug delivery device 500, e.g., on a size and shape of the track 544, on a size of the vial 518, etc. In general, the larger the inner diameter of the vial 518, the less that the plunger 510 will need to move proximally to cause a sufficient amount of the drug to be sprayed out of the drug delivery device 500. In this illustrated embodiment, the vial 518 has an inner diameter of about 6 mm, and the plunger 510 moves proximally by about 3.6 mm in each actuation.

The biasing force provided by the spring 534 that is needed to cause proximal movement of the vial holder 516 and the vial 518 in each actuation varies depending on a configuration of the drug delivery device 500, e.g., on distances between successive ones of the ratchet steps 538, a combined weight of the vial holder 516, vial 518, and drug contained in the vial 518, etc. In general, the force provided by the spring 534 to cause the proximal movement of the vial holder 516 and the vial 518 decreases with each actuation since there is less drug in the vial 518 after each actuation and thus less weight to be urged proximally. For example, in this illustrated embodiment, the force provided by the spring 534 is configured to decrease by about 36%, e.g., from about 14 N to about 9 N.

The drug delivery device 500 in this illustrated embodiment is not powered, e.g., does not include any electrical components such as a processor, a sensor, a memory, a communications interface, etc. The drug delivery device 500 thus does not need to include a power supply though a power supply could be included, e.g., for connectivity, for powering a light source of the drug delivery device, etc.

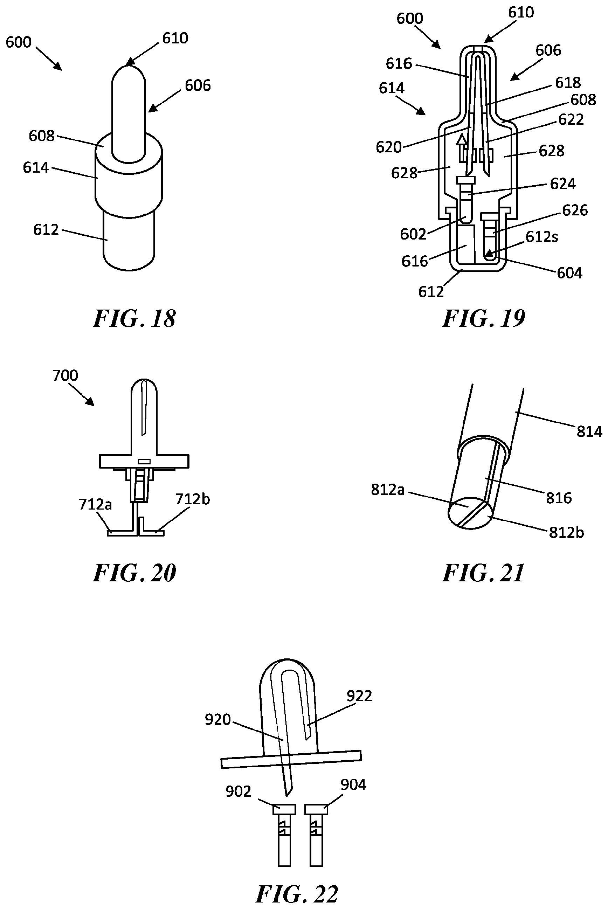

FIGS. 18 and 19 illustrate another exemplary embodiment of the drug delivery device 100 of FIG. 1. FIGS. 18 and 19 illustrate a drug delivery device 600 configured to expel a drug into a nose of a patient and that is generally configured and used similar to that discussed above regarding FIG. 1. The drug delivery device 600 includes first and second vials 602, 604 that each contain enough drug therein for one drug spray. The drug delivery device 600 is thus configured to deliver two drug sprays. In other words, each of the multiple sprays deliverable from the drug delivery device 600 includes drug from the one vial 602, 604. The drug delivery device 600 in this illustrated embodiment is configured to deliver each of the two drug sprays into the same nostril as part of one continuous actuation of the drug delivery device 600. The vials 602, 604 are each generally configured and used similar to that discussed above regarding the vial 200 of FIG. 2.

The drug delivery device 600 includes a dispensing head that includes a tip 606, a depth guide 608, and an opening 610. The tip 606 is configured to be inserted into a nostril to deliver drug into the nostril from each of the vials 602, 604. The opening 610 in this illustrated embodiment is a single opening through which drug from each of the vials 602, 604 is configured to exit the drug delivery device 600. In other embodiments, the opening 610 can include two openings, one opening for drug delivery from each of the vials 602, 604. The drug delivery device 600 includes a body 614 that defines the dispensing head. In other embodiments, the body 614 can be a separate member from the tip 606 and have the tip 606 attached thereto.

The vials 602, 604 are pre-loaded into the drug delivery device 600 during manufacturing. The drug delivery device 600 is thus configured to be provided to an end user with the vials 602, 604 already contained therein.

The drug in each of the vials 602, 604 can be the same as one another, or the drug in each of the vials 602, 604 can be different from the drug in the other one of the vials 602, 604.

The drug delivery device 600 also includes an actuator configured to be actuated to cause drug to exit the drug delivery device 600 through the opening 610. The actuator includes a plunger 612 in this illustrated embodiment. The drug in this illustrated embodiment is configured to be self-administered such that the user who actuates the actuator, e.g., the plunger 612, is the person receiving the drug from the drug delivery device 600, although another person can actuate the device 600 for delivery into another person. The plunger 612 includes a raised platform 616 that extends proximally from a bottom inner surface 612s of the plunger 612 and that is configured to seat the first vial 602 thereon. The second vial 604 is configured to be seated on the bottom inner surface 612s of the plunger 612. The first vial 602 is thus configured to be positioned more proximally than the second vial 604, at least when the drug delivery device 600 is in an initial configuration, which is illustrated in FIGS. 18 and 19.

The drug delivery device 600 includes, for each of the vials 602, 604, a cannula 616, 618 and a pin 620, 622 held by its respective cannula 616, 618. Each cannula 616, 618 includes an inner passageway that is in communication with the opening 610 and with an inner passageway of its associated pin 620, 622. Each pin 620, 622 is seated in a distal portion of the inner passageway of its associated cannula 616, 618 and extends distally from the cannula 616, 618. Each pin 620, 622 is configured to pierce or puncture a seal member 624, 626 of its associated vial 602, 604, as discussed further below. With the pin 620, 622 pierced or punctured through its associated seal member 624, 626, the drug in the associated vial 602, 604 can exit the vial 602, 604, then travel proximally in the inner passageway of the pin 602, 622, then travel proximally in the inner passageway of the associated cannula 616, 618, and then exit the drug delivery device 600 through the opening 610.

The drug delivery device 600 includes a spring 628 operatively coupled to the plunger 612. The spring 628 is a coil spring in this illustrated embodiment but can be another type of spring or biasing member. The spring 628 is configured to bias the plunger 612 in a distal direction. A proximal end of the spring 628 abuts the body 614, e.g., an upper inner surface of the body 614. A distal end of the spring 628 abuts the plunger 612, e.g., a proximal surface of the plunger 612. The spring 334 being fixed at either end thereof to the body 614 and the plunger 612 is configured to allow the plunger 612 to move relative to the body 614.

FIGS. 18 and 19 illustrate the drug delivery device 600 in an initial configuration. The actuation of the plunger 612 includes pushing the plunger 612 in a proximal direction, such as by a user or other element pushing on a distal surface of the plunger 612. The proximal pushing of the plunger 612 causes the plunger 612, including the raised platform 616 thereof, to move proximally relative to the cannulas 616, 618, the pins 620, 622, and the body 614, and thus relative to the dispensing head 630. The vials 602, 604 move proximally with the plunger 612. The first vial 602 is raised proximally relative to the second vial 604 due to the first vial 602 being seated on the platform 616. The first vial 602 thus encounters its associated cannula 616 and pin 620 before the second vial encounters its associated cannula 618 and pin 622. The pushing of the plunger 612 therefore first causes the first pin 620 to pierce or puncture the first seal member 624, thereby causing the first pin's inner passageway to become in fluid communication with the drug in the first vial 602. The drug in the first vial 602 is thus drawn proximally into the first pin's inner passageway, then into the first cannula's inner passageway, and then out the opening 610. A spray chamber of the tip 606 defined by the cannula's inner passageway facilitates the drug exiting the opening 610 as a spray, as discussed above. The plunger 612 continues to move proximally such that after the first spray has been delivered out of the opening 610, the pushing of the plunger 612 causes the second pin 622 to pierce or puncture the second seal member 626, thereby causing the second pin's inner passageway to become in fluid communication with the drug in the second vial 604. The drug in the second vial 604 is thus drawn proximally into the second pin's inner passageway, then into the second cannula's inner passageway, and then out the opening 610. The spring 628 compresses during the proximal movement of the plunger 612.

In response to the proximally directed force being removed from the plunger 612, e.g., from the user or other element ceasing to push proximally on the plunger 612, the spring 628 is allowed to expand and thereby cause the plunger 612 to move distally relative to the body 614 and thus relative to the dispensing head.

In some embodiments, the drug delivery device 600 can include a locking mechanism that locks the plunger 612 in position relative to the body 614 after the two drug sprays have been delivered from the drug delivery device 600. The locking mechanism can lock the plunger 612 in a proximal position relative to the plunger's initial position, which may help indicate that the drug delivery device 600 has been actuated to deliver drug therefrom since the plunger 612 will be in a different position than before actuation. The locking mechanism can have a variety of configurations. For example, one of the body 614 and the plunger 612 can include a flange extending therefrom that is configured to automatically seat in a groove formed in the other of the body 614 and the plunger 612 when the flange and the groove become aligned with one another. For another example, the body 614 can define an internal opening such that the plunger 612 is locked in position relative to the body 614 after a lip of the plunger 612 passes through the opening, similar to the distal opening 342 and the lip 344 of the drug delivery device 300 of FIG. 3.

The drug delivery device 600 in this illustrated embodiment is not powered, e.g., does not include any electrical components such as a processor, a sensor, a memory, a communications interface, etc. The drug delivery device 600 thus does not need to include a power supply though a power supply could be included, e.g., for connectivity, for powering a light source of the drug delivery device, etc.

FIG. 20 illustrates another exemplary embodiment of the drug delivery device 100 of FIG. 1. FIG. 20 illustrates a drug delivery device 700 configured to expel a drug into a nose of a patient and that is generally configured and used similar to that discussed above regarding the drug delivery device 600 of FIGS. 18 and 19. However, the drug delivery device 700 of FIG. 20 includes a split plunger configured to allow for two independent actuations that separately cause drug deliveries from first and second vials of the drug delivery device 700. The split plunger includes a first half 712a configured to be pushed proximally to cause drug delivery from the first vial, and includes a second half 712b configured to be pushed proximally to cause drug delivery from the second vial. The drug delivery device 700 in this illustrated does not include a raised platform like the raised platform 616 of FIG. 19.

The drug delivery device 700 in this illustrated embodiment is not powered, e.g., does not include any electrical components such as a processor, a sensor, a memory, a communications interface, etc. The drug delivery device 700 thus does not need to include a power supply though a power supply could be included, e.g., for connectivity, for powering a light source of the drug delivery device, etc.

FIG. 21 illustrates another exemplary embodiment of a split plunger that includes a first half 812a configured to be pushed proximally relative to the drug delivery device's body 814 to cause drug delivery from a first vial, and includes a second half 812b configured to be pushed proximally relative to the body 814 to cause drug delivery from a second vial. FIG. 21 also illustrates one embodiment of a rib 816 that is positioned between the two plunger halves 812a, 812b and that is configured to prevent both of the plunger halves 812a, 812b from being actuated, e.g., pushed proximally, at a same time.

FIG. 22 illustrates another exemplary embodiment of the drug delivery device 100 of FIG. 1. FIG. 22 illustrates a drug delivery device 900 configured to expel a drug into a nose of a patient and that is generally configured and used similar to that discussed above regarding the drug delivery device 600 of FIGS. 18 and 19. However, the drug delivery device 900 of FIG. 22 does not include a raised platform like the raised platform 616 of FIG. 19. Instead, the drug delivery device 900 is configured to deliver each of two drug sprays from first and second vials 902, 904 into the same nostril as part of one continuous actuation of the drug delivery device 900 by using different length cannulas 920, 922. In this illustrated embodiment, the first cannula 920 has a longer length and extends farther distally than the second cannula 922. The drug in the first vial 902 will thus be accessed by the first cannula 920 (and its associated pin) before the drug in the second vial 904 is accessed by the second cannula 922 (and its associated pin). Instead of the cannulas 920, 924 having different lengths, their associated pins can have different lengths to similarly cause the drug in the first vial 902 to be accessed before the drug in the second vial 904 is accessed.

The drug delivery device 900 in this illustrated embodiment is not powered, e.g., does not include any electrical components such as a processor, a sensor, a memory, a communications interface, etc. The drug delivery device 900 thus does not need to include a power supply though a power supply could be included, e.g., for connectivity, for powering a light source of the drug delivery device, etc.

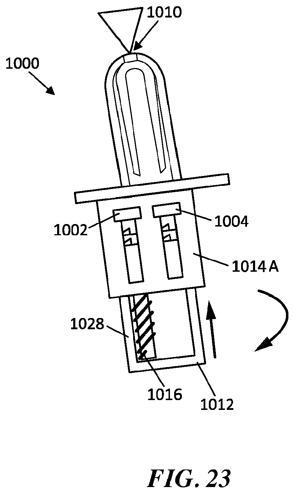

FIG. 23 illustrates another exemplary embodiment of the drug delivery device 100 of FIG. 1. FIG. 23 illustrates a drug delivery device 1000 configured to expel a drug into a nose of a patient and that is generally configured and used similar to that discussed above regarding the drug delivery device 600 of FIGS. 18 and 19. However, while the drug delivery device 1000 of FIG. 23 does include a raised platform 1016 similar to the raised platform 616 of FIG. 19, the drug delivery device 1000 is configured to allow for two independent actuations that separately cause drug deliveries from first and second vials 1002, 1004 of the drug delivery device 1000. **In** this illustrated embodiment, a spring 1028 of the drug delivery device 1000 is configured to return the drug delivery device's plunger 1012 back to its initial position after drug from the first vial 1002 has been sprayed out of the drug delivery device's opening 1010. The plunger 1012, including the platform 1016 thereof, is configured to be rotated relative to the drug delivery device's body 1014 after the first drug spray, as shown by arrow A in FIG. 23. The rotation of the plunger 1012 causes the platform 1016 to be aligned with the second vial 1004. Thus, in response to a second actuation of the plunger 1012, drug is delivered from the second vial 1004. Similar to that discussed above regarding the drug delivery device 600 of FIGS. 18 and 19, the spring 1028 can be configured to move the plunger 1012 distally after the second drug delivery, or the drug delivery device 1000 can include a locking mechanism configured to lock the plunger 1012 in position relative to the body 1014.

The drug delivery device 1000 in this illustrated embodiment is not powered, e.g., does not include any electrical components such as a processor, a sensor, a memory, a communications interface, etc. The drug delivery device 1000 thus does not need to include a power supply though a power supply could be included, e.g., for connectivity, for powering a light source of the drug delivery device, etc.

Embodiments of nasal drug delivery devices disclosed herein can be designed to be disposed of after a single use, or they can be designed to be used multiple times. In either case, in at least some embodiments, the drug delivery device can be reconditioned for reuse after at least one use. Reconditioning can include any combination of the steps of disassembly of the drug delivery device, followed by cleaning or replacement of particular pieces and subsequent reassembly. In particular, the drug delivery device can be disassembled, and any number of the particular pieces or parts of the drug delivery device can be selectively replaced or removed in any combination. Upon cleaning and/or replacement of particular parts, the drug delivery device can be reassembled for subsequent use either at a reconditioning facility, or by a health care provider immediately prior to use. A person skilled in the art will appreciate that reconditioning of a drug delivery device can utilize a variety of techniques for disassembly, cleaning/replacement, and reassembly. Use of such techniques, and the resulting reconditioned drug delivery device, are all within the scope of the present application.

The present disclosure has been described above by way of example only within the context of the overall disclosure provided herein. It will be appreciated that modifications within the scope of the claims may be made without departing from the overall scope of the present disclosure.

## Claims

1. A drug delivery device (300), comprising:
a tip (304) configured to be positioned in a nose of a patient, the tip (304) having an opening (308) therein;
a cartridge (316) configured to seat a plurality of vials (318) that each contain a drug therein, a first one of the vials (318) being aligned with the tip (304); and
a plunger (310) configured to be actuated to cause drug in a first one of the vials (318) to be delivered through the opening (308) **characterized in that** the plunger is further configured to be actuated to cause the cartridge (316) to rotate relative to the tip (304) and to the plunger (310) such that the first one of the vials (318) is misaligned from the tip and a second one of the vials (318) is aligned with the tip (304).

2. The device of claim 1, wherein the plurality of vials (318) includes a total of six vials.

3. The device of claim 1, wherein the plurality of vials (318) includes only the first and second vials.

4. The device of any one of the preceding claims, wherein the actuation of the plunger (310) includes moving the plunger (310) proximally relative to the tip (304); and
the drug delivery device further comprises a spring (334) configured to compress during the proximal movement of the tip (304) and to automatically expand to cause the plunger (310) to move distally relative to the tip (304).

5. The device of any one of the preceding claims, further comprising a body (312) that includes a protrusion (350);
wherein the cartridge (316) has a groove (348) formed therein in which the protrusion (350) is configured to slide to cause the rotation of the cartridge (316) about a longitudinal axis of the cartridge.

6. The device of claim 5, wherein the actuation of the plunger (310) includes moving the plunger (310) longitudinally relative to the tip (304) and the body (312).

7. The device of claim 5 or claim 6, wherein a longitudinal axis of the tip (304) is offset from the longitudinal axis of the cartridge (316).

8. The device of any one of claims 5 to 7, wherein the body includes a window (322) therein, and the cartridge (316) includes a plurality of indicators (320) thereon that are configured to be visible sequentially through the window (322).

9. The device of claim 8, wherein the rotation of the cartridge (316) causes a one of the indictors (320) that is visible through the window (322) to change to another one of the indicators (320).

10. The device of any one of the preceding claims, wherein, with the second one of the vials (318) aligned with the tip (304), the plunger (310) is configured to be actuated again to cause drug in the second one of the vials (318) to be delivered through the opening (304) and to cause the cartridge (316) to rotate relative to the tip (304) and to the plunger (310).

11. The device of claim 10, wherein the rotation of the cartridge (316) causes the second one of the vials (318) to become misaligned from the tip (304) and a third one of the vials (318) to be aligned with the tip (304).

12. The device of claim 10, wherein the plunger (310) cannot be actuated again after the drug in the second one of the vials (318) is delivered through the opening (308).

13. The device of any one of the preceding claims, further comprising the plurality of vials (318) that each contain the drug therein.

14. The device of any one of the preceding claims, wherein the drug in each of the vials is one of ketamine, esketamine, naloxone, and sumatriptan.

## Patentansprüche

1. Arzneimittelabgabevorrichtung (300), umfassend:
eine Spitze (304), die konfiguriert ist, um in einer Nase eines Patienten positioniert zu werden, wobei die Spitze (304) eine Öffnung (308) darin aufweist;
eine Kartusche (316), die konfiguriert ist, um eine Vielzahl von Fläschchen (318) aufzunehmen, die jeweils ein Arzneimittel darin enthalten, wobei ein erstes der Fläschchen (318) mit der Spitze (304) ausgerichtet ist; und
einen Kolben (310), der konfiguriert ist, um betätigt zu werden, um zu bewirken, dass das Arzneimittel in einem ersten der Fläschchen (318) durch die Öffnung (308) abgegeben wird, **dadurch gekennzeichnet, dass** der Kolben ferner konfiguriert ist, um betätigt zu werden, um zu bewirken, dass sich die Kartusche (316) relativ zu der Spitze (304) und zu dem Kolben (310) dreht, sodass das erste der Fläschchen (318) gegenüber der Spitze falsch ausgerichtet ist und ein zweites der Fläschchen (318) mit der Spitze (304) ausgerichtet ist.

2. Vorrichtung nach Anspruch 1, wobei die Vielzahl von Fläschchen (318) insgesamt sechs Fläschchen einschließt.

3. Vorrichtung nach Anspruch 1, wobei die Vielzahl von Fläschchen (318) nur das erste und das zweite Fläschchen einschließt.

4. Vorrichtung nach einem der vorstehenden Ansprüche, wobei die Betätigung des Kolbens (310) ein proximales Bewegen des Kolbens (310) relativ zu der Spitze (304) einschließt; und
die Arzneimittelabgabevorrichtung ferner eine Feder (334) umfasst, die konfiguriert ist, um sich während der proximalen Bewegung der Spitze (304) zusammenzudrücken und um sich automatisch auszudehnen, um zu bewirken, dass sich der Kolben (310) relativ zu der Spitze (304) distal bewegt.

5. Vorrichtung nach einem der vorstehenden Ansprüche, ferner umfassend einen Körper (312), der einen Vorsprung (350) einschließt;
wobei die Kartusche (316) eine darin ausgebildete Nut (348) aufweist, in der der Vorsprung (350) konfiguriert ist, um die Drehung der Kartusche (316) um eine Längsachse der Kartusche herum zu bewirken.

6. Vorrichtung nach Anspruch 5, wobei die Betätigung des Kolbens (310) ein longitudinales Bewegen des Kolbens (310) relativ zu der Spitze (304) und dem Körper (312) einschließt.

7. Vorrichtung nach Anspruch 5 oder 6, wobei eine Längsachse der Spitze (304) gegenüber der Längsachse der Kartusche (316) versetzt ist.

8. Vorrichtung nach einem der Ansprüche 5 bis 7, wobei der Körper ein Fenster (322) darin einschließt und die Kartusche (316) eine Vielzahl von Anzeigen (320) darauf einschließt, die konfiguriert sind, um durch das Fenster (322) sequenziell sichtbar zu sein.

9. Vorrichtung nach Anspruch 8, wobei die Drehung der Kartusche (316) bewirkt, dass sich eine der Anzeigen (320), die durch das Fenster (322) sichtbar ist, zu einer anderen der Anzeigen (320) ändert.

10. Vorrichtung nach einem der vorstehenden Ansprüche, wobei, wenn die zweite der Fläschchen (318) mit der Spitze (304) ausgerichtet ist, der Kolben (310) konfiguriert ist, um erneut betätigt zu werden, um zu bewirken, dass das Arzneimittel in dem zweiten der Fläschchen (318) durch die Öffnung (304) abgegeben wird und um zu bewirken, dass sich die Kartusche (316) relativ zu der Spitze (304) und zu dem Kolben (310) dreht.

11. Vorrichtung nach Anspruch 10, wobei die Drehung der Kartusche (316) bewirkt, dass das zweite der Fläschchen (318) gegenüber der Spitze (304) falsch ausgerichtet wird und ein drittes der Fläschchen (318) mit der Spitze (304) ausgerichtet ist.

12. Vorrichtung nach Anspruch 10, wobei der Kolben (310) nicht erneut betätigt werden kann, nachdem das Arzneimittel in dem zweiten der Fläschchen (318) durch die Öffnung (308) abgegeben wurde.

13. Vorrichtung nach einem der vorstehenden Ansprüche, ferner umfassend die Vielzahl von Fläschchen (318), die jeweils das Arzneimittel darin enthalten.

14. Vorrichtung nach einem der vorstehenden Ansprüche, wobei das Arzneimittel in jedem der Fläschchen eines von Ketamin, Esketamin, Naloxon oder Sumatriptan ist.

## Revendications

1. Dispositif d'administration de médicament (300), comprenant :
un embout (304) conçu pour être positionné dans le nez d'un patient, l'embout (304) ayant une ouverture (308) à l'intérieur de celui-ci ;
une cartouche (316) conçue pour loger une pluralité de flacons (318) qui contiennent chacun un médicament à l'intérieur de ceux-ci, un premier des flacons (318) étant aligné avec l'embout (304) ; et
un piston (310) conçu pour être actionné pour provoquer l'administration du médicament dans un premier des flacons (318) à travers l'ouverture (308), **caractérisé en ce que** le piston est en outre conçu pour être actionné pour amener la cartouche (316) à tourner par rapport à l'embout (304) et au piston (310) de telle sorte que le premier des flacons (318) est désaligné par rapport à l'embout et qu'un deuxième des flacons (318) est aligné avec l'embout (304).

2. Dispositif selon la revendication 1, dans lequel la pluralité de flacons (318) comportent un total de six flacons.

3. Dispositif selon la revendication 1, dans lequel la pluralité de flacons (318) comportent uniquement les premier et deuxième flacons.

4. Dispositif selon l'une quelconque des revendications précédentes, dans lequel l'actionnement du piston (310) comporte le déplacement du piston (310) de manière proximale par rapport à l'embout (304) ; et
le dispositif d'administration de médicament comprend en outre un ressort (334) conçu pour se comprimer pendant le mouvement proximal de l'embout (304) et pour se détendre automatiquement pour amener le piston (310) à se déplacer de manière distale par rapport à l'embout (304).

5. Dispositif selon l'une quelconque des revendications précédentes, comprenant en outre un corps (312) qui comporte une saillie (350) ;
dans lequel la cartouche (316) a une rainure (348) formée dans celle-ci, dans laquelle la saillie (350) est conçue pour coulisser afin de provoquer la rotation de la cartouche (316) autour d'un axe longitudinal de la cartouche.

6. Dispositif selon la revendication 5, dans lequel l'actionnement du piston (310) comporte le déplacement du piston (310) longitudinalement par rapport à l'embout (304) et au corps (312).

7. Dispositif selon la revendication 5 ou la revendication 6, dans lequel un axe longitudinal de l'embout (304) est décalé par rapport à l'axe longitudinal de la cartouche (316).

8. Dispositif selon l'une quelconque des revendications 5 à 7, dans lequel le corps comporte une fenêtre (322) à l'intérieur de celui-ci, et la cartouche (316) comporte une pluralité d'indicateurs (320) sur celle-ci qui sont conçus pour être visibles séquentiellement à travers la fenêtre (322).

9. Dispositif selon la revendication 8, dans lequel la rotation de la cartouche (316) amène l'un des indicateurs (320) qui est visible à travers la fenêtre (322) à changer pour un autre des indicateurs (320).

10. Dispositif selon l'une quelconque des revendications précédentes, dans lequel, lorsque le deuxième des flacons (318) est aligné avec l'embout (304), le piston (310) est conçu pour être actionné à nouveau afin d'amener le médicament dans le deuxième des flacons (318) à être administré à travers l'ouverture (304) et d'amener la cartouche (316) à tourner par rapport à l'embout (304) et au piston (310).

11. Dispositif selon la revendication 10, dans lequel la rotation de la cartouche (316) amène le deuxième des flacons (318) à se désaligner de l'embout (304) et un troisième des flacons (318) à s'aligner avec l'embout (304).

12. Dispositif selon la revendication 10, dans lequel le piston (310) ne peut pas être actionné à nouveau après que le médicament dans le deuxième des flacons (318) a été administré à travers l'ouverture (308).

13. Dispositif selon l'une quelconque des revendications précédentes, comprenant en outre la pluralité de flacons (318) qui contiennent chacun le médicament à l'intérieur de ceux-ci.

14. Dispositif selon l'une quelconque des revendications précédentes, dans lequel le médicament dans chacun des flacons est l'un parmi kétamine, eskétamine, naloxone et sumatriptan.
